# EUROPEAN PATENT SPECIFICATION

(11) **EP 3 013 372 B1**
(45) Date of publication and mention of the grant of the patent: **10.07.2019**
(21) Application number: 15803069.2
(22) Date of filing: 02.06.2015
(51) Int. Cl.: A61K 49/00, G01N 33/84, A61K 31/192, A61K 31/25, A61K 31/216

(54) **METHODS FOR TREATING UREA CYCLE DISORDERS TO PREVENT HYPERAMMONEMIC CRISES BY CONTROLLING BLOOD AMMONIA LEVELS**
VERFAHREN ZUR BEHANDLUNG VON HARNSTOFFZYKLUSERKRANKUNGEN ZUR VERHINDERUNG HYPERAMMONÄMISCHER KRISEN DURCH REGELUNG DES AMMONIAKSPIEGELS IM BLUT
PROCÉDÉS POUR LE TRAITEMENT DES TROUBLES DU CYCLE DE L'URÉE AFIN DE PRÉVENIR LES CRISES D'HYPERAMMONIÉMIE PAR RÉGULATION DU TAUX D'AMMONIAC DANS LE SANG

(30) Priority: 04.06.2014 US 201462007894 P
(43) Date of publication of application: 04.05.2016
(73) Proprietor: Immedica Pharma AB, 113 29 Stockholm (SE)
(72) Inventor: SCHARSCHMIDT, Bruce, San Francisco, CA 94127 (US); MOKHTARANI, Masoud, Walnut Creek, CA 94598 (US)
(74) Representative: Zacco Sweden AB
(86) International application number: PCT/US2015/033700
(87) International publication number: WO 2015/187641

(56) References cited:
- WO-A1-2009/134460
- US-A1- 2013 085 179
- US-A1- 2013 281 530
- Anomynous: "Application Number 20-645 Medical Review FDA", , 15 February 2005 (2005-02-15), pages 1-55, XP055260195, Retrieved from the Internet: URL:http://www.accessdata.fda.gov/drugsatf da_docs/nda/2005/020645s000_MedR.pdf [retrieved on 2016-03-22]
- South San ET AL: "Hyperion Therapeutics Announces Presentation of Long Term Data on Ammonia Control in Pediatric Patients Treated With RAVICTI(R) at the 12th International Congress of Inborn Errors of Metabolism and the Urea Cycle Disorder Satellite Symposium", , 3 September 2013 (2013-09-03), XP055260208, Retrieved from the Internet: URL:http://files.shareholder.com/downloads /AMDA-1412CE/0x0x688110/4e684e9d-6c54-4963 -a993-72c90f308802/HPTX_News_2013_9_3_Gene ral_Releases.pdf [retrieved on 2016-03-21]
- BRENDAN LEE ET AL: "Blood ammonia and glutamine as predictors of hyperammonemic crises in patients with urea cycle disorder", GENETICS IN MEDICINE, vol. 17, no. 7, 11 December 2014 (2014-12-11), pages 561-568, XP055260189, US ISSN: 1098-3600, DOI: 10.1038/gim.2014.148

## Description

Urea cycle disorders (UCDs) are inborn errors of metabolism involving deficiencies of enzymes required for ureagenesis. They are manifested by acute and chronic hyperammonemia and medical management is aimed at reducing waste nitrogen through the restriction of protein intake and the use of alternate pathway drugs, historically sodium phenylbutyrate (NaPBA, also referred to by the US trade name BUPHENYL® and the EU trade name AMMONAPS®) and more recently glycerol phenylbutyrate (GPB; also referred to as RAVICTI® or HPN-100). UCDs include several inherited deficiencies of enzymes or transporters necessary for the synthesis of urea from ammonia, including enzymes involved in the urea cycle.

Most UCD patients are at a lifelong risk of hyperammonemic crises (HAC), sometimes several per year, in which ammonia levels rise in their blood and brain, and cause symptoms ranging from nausea, vomiting, and headache to coma and death (Haberle 2011). These crises can be triggered by infections, postsurgery, or pregnancy (Haberle 2012).

NaPBA and GPB are ammonia-lowering agents that have been approved for the management of high blood levels of ammonia caused by UCDs not manageable by diet alone. Control of blood ammonia is a central management objective for UCD patients, but current guidelines do not specify the optimal target levels of ammonia to prevent HACs (Berry 2001). More information is needed to understand how ammonia levels relate to outcome in UCD patients to identify the optimal target levels of ammonia to treat UCDs and prevent HACs for optimal treatment management. Thus, there is a need in the art for improved methods for achieving ammonia control and fewer HACs in patients with UCDs. FDA Medical Review Application Number 20-645 discusses the drug "Ammonul" (sodium phenylacetate and sodium benzoate 10%/10% for injection) which is indicated for the treatment of acute hyperammonemia in patients with UCDs. "Hyperion Therapeutics Announces Presentation of Long Term Data on Ammonia Control in Pediatric Patients Treated with RAVICTI® at the 12th International Congress of Inborn Errors of Metabolism and the Urea Cycle Disorder Satellite Symposium" (3rd September 2013) highlights long term data regarding ammonia control in pediatric patients with UCDs who were treated with RAVICTI® oral liquid. US 2013/0085179 A1 discloses methods for evaluating daily ammonia exposure based on a single fasting ammonia blood level measurement, as well as methods that utilize this technique to adjust the dosage of a nitrogen scavenging drug, determine whether to administer a nitrogen scavenging drug, and treat nitrogen retention disorders.

The invention, which is defined in the appended claims, provides GPB for use in novel methods for preventing an HAC in a subject with a UCD who has been previously treated with a nitrogen scavenging drug and who has a glutamine level greater than 649 µmοl/L. These methods comprise (a) measuring a fasting blood ammonia level; (b) comparing the fasting blood ammonia level to the upper limit of normal (ULN) for blood ammonia; and (c) administering an effective dose of GPB to the subject if the fasting blood ammonia level is above 0.5 times the ULN for fasting blood ammonia. In these methods, the effective dosage of GPB results in the fasting blood ammonia level being <0.5 times the ULN for blood ammonia. In these methods, the subject is aged 6 years or older. In these methods, the nitrogen scavenging drug is selected from the group consisting of sodium benzoate, GPB, phenylbutyric acid (PBA), NaPBA, and a combination of two or more of GPB, PBA, and NaPBA In certain embodiments, the subject has previously been administered a first dosage of GPB. In certain embodiments, the dosage of GPB administered in step (c) is greater than the first dosage. In certain embodiments, the HAC is a first HAC. In certain embodiments, the subject has been treated with GPB In certain embodiments, the subject has been previously treated with GPB for an amount of time to maintain a steady state level of the drug in the subject.

The PAA prodrug used in the invention is GPB.

### BRIEF DESCRIPTION OF DRAWINGS

FIG. 1: A boxplot of baseline ammonia for ULN baseline categories, < 0.5 ULN, 0.5 to < 1.0 ULN, and ≥ 1.0 ULN.
FIG. 2: A bar graph showing the percentage of patients whom experienced an HAC as categorical baseline ammonia increased.
FIG. 3: A bar graph of HAC and non HAC events between baseline ammonia quartiles.
FIG. 4: A bar graph showing the percentage of patients with no HAC and the percentage of patients whom experienced an HAC across different baseline ammonia level categories. Black bars represent patients whom experienced an HAC and gray bars represent patients with no HAC.
FIG. 5: A Kaplan-Meier (KM) plot of time to first HAC.
FIG. 6: A KM plot of HAC-free survival in relation to baseline ammonia categorized in relation to ULN. Log-rank test p-value=0.0077. The open circle represents < 0.5 ULN data, the open square represents 0.5 to < 1.0 ULN data, and the open triangle represents ≥ 1.0 ULN data.
FIG. 7: A bar graph showing HAC incidence rate ratios based on negative binomial regression for baseline ammonia ULN categories.
FIG. 8: A histogram of duration of HAC in days. Note the steep drop off in the distribution after two days.
FIG. 9: A KM plot of time to first HAC by age categories. The open circle represents data from patients ages 11-18 years old (N=9); the open square represents data from patients age 18 and older (N=51); the open triangle represents data from patients ages 6-11 years old (N=17); and the black circle represents data from patients less than 6 years old (N=23).
FIG. 10: A KM plot of time to first HAC by binary age category (adult vs. pediatric). The open circle represents data from patients 18 years and older (N=51) and the open triangle represents data from patients less than 18 years old (N=49).
FIG. 11: KM plots of time to first HAC in subjects 6 years of age and older categorized by ULN baseline ammonia category. Log-rank test p-value=0.0058. The open circle represents 0 to < 0.5 ULN data, the open triangle represents 0.5 to < 1.0 ULN data, and the open square represents ≥ 1.0 ULN data.
FIG. 12: A bar graph showing relative risk of HAC by incremental increase in ammonia exposure. 1.50 indicates 50% increased risk of having HAC with each 10 units ammonia increase relative to patients with no increase in ammonia.

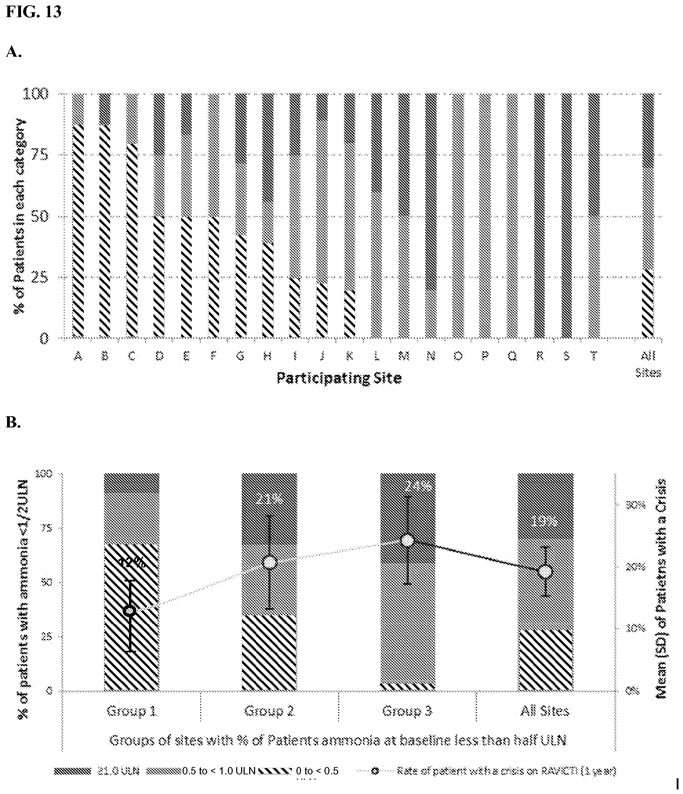

FIG. 13: Ammonia levels and HAC rate by study site. A: A graph shows the percentage of patients at each study site with baseline ammonia <0.5 ULN (black and white stripes), 0.5 - < 1.0 ULN (light gray), and ≥ 1.0 ULN (dark gray). B: A bar graph shows the percentage of patients in each ammonia ULN category by sites grouped in relation to ammonia levels among patients enrolled at the site. Group 1 (6 study sites; 33 patients, of whom >50% had baseline ammonia levels < 0.5 ULN); Group 2 (3 study sites; 29 patients, of whom 25%-50% had baseline ammonia levels < 0.5 ULN); and Group 3 (11 sites; 38 patients, of whom < 25% had baseline ammonia < 0.5 ULN). The black and white striped bar represents 0 to < 0.5 ULN data, the light gray bar represents 0.5 to < 1.0 ULN data, and the dark gray bar represents ≥ 1.0 ULN data.

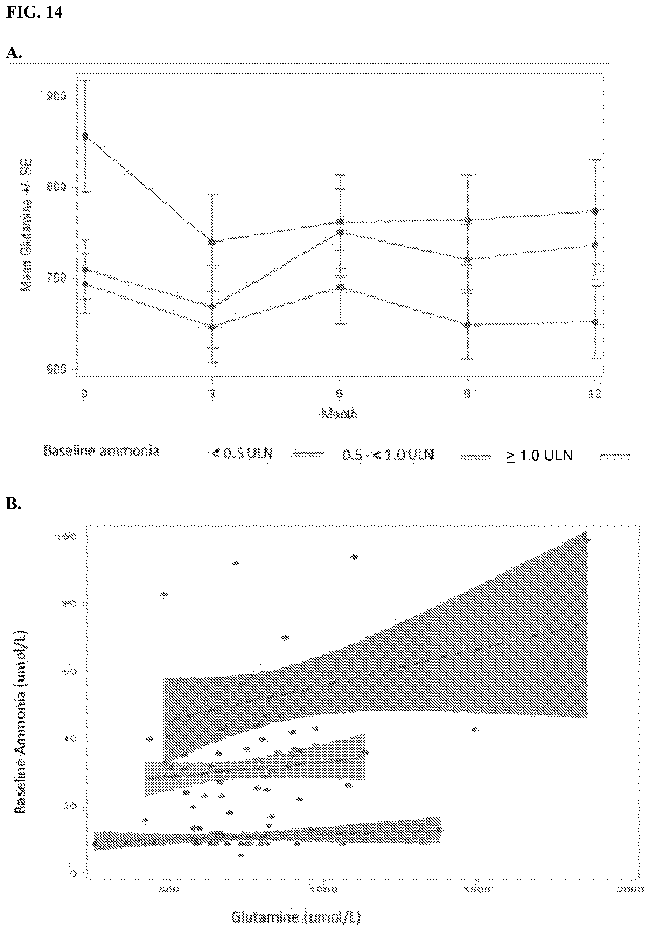

FIG. 14: Relationship between glutamine and ammonia levels by baseline ammonia categories. A: A line graph showing glutamine levels during 12-months of treatment with GPB. The mean (± SE) monthly glutamine levels (y-axis) for baseline ammonia ULN categories are shown for months 0, 3, 6, 9, and 12 (x-axis). The top line represents data from patients with ≥ 1.0 ULN, the middle line represents data from patients with 0.5 to < 1.0 ULN, and the bottom line represents data from patients with < 0.5 ULN. B: Scatterplot of baseline glutamine by ammonia ULN categories. The overall correlation between glutamine and ammonia was significant (r = 0.27; p = 0.008), whereas none of the correlations within ammonia ULN categories was significant (r=0.12; 0.18 and 0.12 respectively; p=NS for all). The top line represents data from patients with ≥ 1.0 ULN, the middle line represents data from patients with 0.5 to < 1.0 ULN, and the bottom line represents data from patients with < 0.5 ULN.
FIG. 15: A box plot of glutamine at baseline by categories of ULN baseline ammonia.
FIG. 16: Relationship between glutamine and ammonia levels by UCD subtype. There was a strong correlation between ammonia and glutamine with proximal defects (OTC, CPS), but not distal defects (ASL, ASS).

The following description of the invention is merely intended to illustrate various embodiments of the invention, which is defined in the appended claims.

Medical management of UCDs is aimed at reducing waste nitrogen through the restriction of protein intake and the use of prodrugs such as phenylbutyrate.
GPB (also referred to as RAVICTI® or HPN-100) is an ammonia-lowering agent that was approved on February 1, 2013 in the United States for use as a nitrogen-binding agent for chronic management of adult and pediatric patients ≥2 years of age with UCDs that cannot be managed by dietary protein restriction and/or amino acid supplementation alone. Current UCD treatment guidelines indicate that ammonia should be kept within normal limits but otherwise provide little guidance on the specific target levels or quantifies the risk of HAC based on ammonia exposure (Berry 2001). As set forth in the examples below, long-term safety studies evaluated the occurrence of HACs during treatment with GPB for up to 1 year. The purpose of the analyses provided herein was to assess the predictive value of blood ammonia in the evaluation of long-term outcomes. Additionally, the utility of glutamine as a predictor of HACs was also assessed.

To identify the optimal target level for ammonia in UCD patients and to assess the long term effects of tight ammonia control, post-hoc statistical analyses were performed as provided herein to interrogate clinical trial data obtained from patients treated for 12 months with GPB. The objective of these analyses was to examine the relationship between fasting ammonia levels and HACs in patients treated with GPB for 12 months. The examples provided herein demonstrate that tight ammonia control results in lower systemic ammonia exposure and better outcome in terms of lower rate of HAC. For example, the findings herein demonstrate that ammonia is a reliable surrogate for assessing adequacy of disease control, as it correlates
strongly with HACs. Additionally, even mildly elevated ammonia over long periods of time may put patients at risk of HAC, and UCD patients, especially those ages 6 years and above, benefit from maintaining fasting ammonia less than 0.5 ULN. Further, fasting ammonia levels less than 0.5 ULN are associated with a lower likelihood of experiencing HACs in the long term.

Data from four short-term switchover (SO) studies and three 12-month safety extension (SE) studies in adult and pediatric UCD patients were included in the analyses; all have been previously described. Protocols UP1204-003, HPN-100-005SO, and HPN-100-012SO were short-term, open-label, fixed sequence SO studies that evaluated ammonia control during equivalent dosing of GPB versus NaPBA (Lee 2010), (Lichter-Konecki 2011), (Smith 2013). Study HPN-100-006 was a pivotal, randomized, double-blind, active-controlled, crossover study that established non-inferiority of GPB to NaPBA as assessed by venous ammonia (Diaz 2013). Most patients completing these protocols plus additional patients (a total of 100 of whom 49 were pediatric) were enrolled into safety extension studies and received GPB for 12 months (Diaz 2013), (Mokhatarani 2013), (Berry 2014).

All protocols and informed consents were reviewed and approved by the Investigational Review Board of each participating institution prior to initiation. Eligible subjects had a confirmed or clinically suspected UCD and had been receiving NaPBA prior to enrollment. In all studies, patients received GPB three times daily (or more frequently in small children to match their prior NaPBA schedule) at a daily dose equivalent to their previously prescribed NaPBA dose. Data regarding HACs were collected for up to 12 months pre-enrollment while patients were on NaPBA, and also during the 12 months of GPB treatment. Fasting ammonia and glutamine levels were analyzed as a predictor of HACs during long-term treatment.

For the long-term studies provided herein, the relationship between ammonia levels and cumulative HAC, time to first HAC, duration of HAC, and relative risk of HAC was analyzed. Age was also analyzed as a predictor of HAC. Since younger children are fed more frequently and obtaining fasting ammonia is often not as practical as it would be for older children, supplemental analyses were performed to examine the rate of HAC and time to first HAC in patients at least 6 years old at baseline. Further analyses were performed to evaluate glutamine levels during the 12 months of GPB treatment.

During the 12 month studies, blood samples for ammonia and glutamine analyses were collected monthly and information on HACs was recorded. Baseline ammonia was defined as the screening or month 0 value when the patient was on NaPBA prior to receiving GPB. An HAC was defined as compatible clinical symptoms associated with one or more ammonia levels ≥ 100 µmol/L. Ammonia and HAC data were also collected retrospectively for up to 12 months prior to enrollment in each study while patients were receiving NaPBA. Ammonia and glutamine concentrations were measured by an accredited hospital laboratory at each study site and ammonia values were normalized to a standard range of 9 to 35 µmol/L.

Fasting ammonia values were divided into three categories: 0 to < 0.5, 0.5 to < 1.0, and ≥ 1.0 relative to the ULN. Except for fasting ammonia, all other baseline characteristics such as age, gender, UCD subtypes, and glutamine were similar across these three groups, although there was a slightly higher proportion of patients aged 6 to 11 years in the 0.5 to < 1.0 ULN group than in the other two groups.

As set forth in the examples below, the relationship between fasting ammonia levels and the likelihood of patients experiencing an HAC was evaluated in UCD patients. It was found that increased fasting ammonia levels correlated strongly with the likelihood of experiencing an HAC. The relationship between ammonia levels and cumulative rate of HAC, time to first HAC, duration of HAC, and relative risk of HAC was analyzed. Based on these results, a comparison of fasting ammonia levels to a specified threshold or target range with respect to the ULN for blood ammonia represents a novel, clinically useful, and practical predictor of HAC risk.

The present application provides practical applications of this finding in the form of GPB for use in methods for preventing an HAC in a subject with UCD. Also disclosed herein is: treating a UCD to prevent an HAC, optimally administering a nitrogen scavenging drug for the treatment of a UCD to prevent an HAC, adjusting and optimizing the dosage of a nitrogen scavenging drug for the treatment of a UCD to prevent an HAC, evaluating the efficacy of a nitrogen scavenging drug for the treatment of a UCD to prevent an HAC, determining whether to administer a nitrogen scavenging drug for the treatment of a UCD to prevent an HAC, minimizing the risk of HACs, predicting the likelihood or risk of an HAC, evaluating and monitoring ammonia exposure, and other related embodiments.

Provided herein are threshold levels and target ranges for fasting blood ammonia upon which an effective dosage of GPB for the treatment of a UCD to prevent HACs can be based. An effective dosage of GPB as used herein refers to a dosage that results in a fasting blood ammonia level falling at or below a specified threshold level or within a specified target range after one or more administrations. In certain embodiments, the effective dosage results in a fasting blood ammonia level falling at or below a specified threshold level or within a specified target range after multiple administrations, and in certain of these embodiments the effective dosage results in a fasting blood ammonia level falling at or below a specified threshold level or within a specified target range after the drug has reached steady state. In certain embodiments, steady state for a particular dosage of GPB is reached at around three days after the initial administration of that dosage. In other embodiments, steady state may be reached at two, three, four, or five days after the initial administration.

Threshold levels and target ranges for fasting blood ammonia are based on the ULN for blood ammonia. In the invention, the specified target range for fasting blood ammonia is <0.5 times the ULN for blood ammonia. The specified threshold level is <0.5 times the ULN for blood ammonia. In certain disclosures a fasting blood ammonia level that is at or above the specified threshold level or above the specified target range indicates that a subject needs to be administered GPB or, where the subject has received a nitrogen scavenging drug previously, that the subject needs to be administered a different nitrogen scavenging drug or a higher dosage of the original nitrogen scavenging drug. Similarly, in certain embodiments a fasting blood ammonia level that is at or below the specified threshold level or within the specified target range indicates that the subject does not need to be administered GPB or, where the subject has received GPB previously, that the subject should continue to be administered GPB and/or the same dosage. In certain embodiments, the optimal range for fasting blood ammonia includes the specified threshold level. In these embodiments, a fasting blood ammonia level at or below the specified threshold level is considered acceptable or optimal.. An effective dosage of GPB may be an initial dosage, subsequent/maintenance dosage, improved dosage, or a dosage determined in combination with other factors. In certain embodiments, the effective dosage may be the same as or different than an initial dosage. In other embodiments, the effective dosage may be higher or lower than an initial dosage.

Disclosed herein are methods of treating a UCD to prevent an HAC in a subject in need thereof. The terms "prevent," "preventing," and "prevention" as used herein with regard to HACs may refer to averting an HAC entirely, reducing the number or frequency of an HAC, decreasing the likelihood of experiencing an HAC, averting, delaying, reducing, or ending symptoms associated with an HAC, or some combination thereof. The terms "treat," "treating," and "treatment" as used herein with regard to UCDs may refer to averting a UCD, slowing the onset or rate of development of a UCD, reducing the risk of developing a UCD, preventing or delaying the development of symptoms associated with a UCD, reducing or ending symptoms associated with a UCD, generating a complete or partial regression of a UCD, or some combination thereof.

As used herein, the term "UCD" refers to urea cycle disorders which are inborn errors of metabolism involving deficiencies of enzymes and/or mitochondrial transporters required for ureagenesis. UCDs are manifested by acute and chronic hyperammonemia.

As used herein, the term "HAC" refers to an episode of high ammonia associated with clinical symptoms such as vomiting, nausea, somnolence and confusion that requires immediate intervention aiming at reducing blood ammonia.

A "subject in need thereof' as used herein refers to a human subject who is prone to or deemed at risk of experiencing an HAC based on one or more genetic and/or environmental factors, or a subject who has previously experienced or is suspected of having experienced an HAC. In certain embodiments, the subject has been previously diagnosed with a UCD, is currently experiencing or suspected of having a UCD, is prone to or has been deemed at risk of experiencing an HAC.

The phrases "patient" and "subject" are used interchangeably herein.

The term "about" as used herein means within 10% of a stated value or range of values.

The fasting ammonia categories used herein are: 0 to < 0.5 times the ULN (or 0 to < 0.5 ULN or < 0.5 ULN), 0.5 to < 1.0 times the ULN (or 0.5 to < 1.0 ULN), and ≥ 1.0 times the ULN (or ≥ 1.0 ULN).

The subject has been previously treated with one or more nitrogen scavenging drugs. In certain embodiments, the subject has been previously treated with NaPBA. In certain embodiments, the subject has been previously treated with NaPBA, but has not been treated with GPB. In certain embodiments, the subject has been treated with one or more nitrogen scavenging drugs. In certain embodiments, the subject has been treated with one or more nitrogen scavenging drugs for an amount of time to maintain a steady state level of the drug in the subject. For example, in certain embodiments, the subject has been treated with one or more nitrogen scavenging drug for at least 3, 4 or 5 days. In certain embodiments, the subject has been treated with one or more nitrogen scavenging drugs for about one year. In certain embodiments, the subject has been treated with GPB.

As shown in Example 1 below, ammonia levels as a function of ULN can be a useful predictor of the risk of experiencing an HAC. For example, the association between ULN categories and HAC was significant so that the percentage of patients who experienced an HAC was lowest in those with a baseline ammonia of less than 0.5 ULN and increased in a step-wise fashion to those with less than 1.0 ULN and was highest in patients with the greater than or equal to 1.0 ULN ammonia value at baseline (p=0.024). In certain embodiments, for a subject that has been previously treated with a nitrogen scavenging drug, the subject's risk of experiencing an HAC may be about 10% if the subject's fasting blood ammonia level is less than 0.5 ULN; about 15% if the subject's fasting blood ammonia level is between 0.5 to less than 1.0 ULN; or about 37% if the subject's fasting blood ammonia level is greater than or equal to 1.0 ULN. In certain embodiments, the subject has been previously treated with GPB, for an amount of time to maintain a steady state level of GPB in the subject. For example, the subject may have been treated with a GPB for at least 3, 4, or 5 days. In some embodiments, the subject has been treated with GPB for about one year. In certain embodiments, for a subject that has been treated with GPB, but has been treated with other nitrogen scavengers such as NaPBA, the subject's risk of experiencing an HAC is about 23% if the subject's fasting blood ammonia level is less than 0.5 ULN; about 26% if the subject's fasting blood ammonia level is between 0.5 to less than 1.0 ULN; or about 44% if the subject's fasting blood ammonia level is greater than or equal to 1.0 ULN. In certain embodiments, the subject may have been previously treated with NaPBA.

As shown in Example 2 below, ammonia levels as a function of ULN can also be a useful predictor of the risk of experiencing a first HAC for a subject. Subjects with higher baseline ammonia levels relative to ULN were found to be at greater risk of HAC compared to patients in lower ULN categories. In certain disclosures, for a subject that has been previously treated with a nitrogen scavenging drug and has a fasting blood ammonia level of greater than or equal to 1.0 ULN, the subject has about a 4.5 times higher risk of experiencing a first HAC compared to subjects with a fasting blood ammonia level of less than 0.5 ULN. In certain disclosures, after adjusting for age, gender and race, for a subject that has been treated with a nitrogen scavenging GPB and has a fasting blood ammonia level of greater than or equal to 1.0 ULN, the subject has about a 5.7 times higher risk of experiencing a first HAC compared to subjects with a fasting blood ammonia level of < 0.5 ULN. In certain disclosures, the subject has been previously treated with GPB, for an amount of time to maintain a steady state level of the drug in the subject. For example, the subject has been treated with GPB for at least 3, 4, or 5 days. In some disclosures, the subject has been treated with GPB for about one year.

As shown in Example 3 below, ammonia levels as a function of ULN can be used to predict the relative incidence for HAC over twelve months or per year. Additionally, subjects that were treated with GPB had a roughly 50% lower incidence rate of HAC compared to those subjects not treated with GPB. In certain disclosures, if the subject has not been previously treated with GPB, then the subject's overall relative incidence of HAC per year may be about 0.581. For example, this means that if 100 subjects that were not treated with GPB were followed for one year, the expected number of HACs would be about 58. In certain disclosures, if the subject has not been previously treated with GPB and the subject's fasting blood ammonia level is less than 0.5 ULN, then the subject's relative incidence of HAC per year may be about 0.433. In certain disclosures, if the subject has not been previously treated with GPB and the subject's fasting blood ammonia level is between 0.5 to less than 1.0 ULN, then the subject's relative incidence of HAC per year may be about 0.389. In certain disclosures, if the subject has not been previously treated with GPB and the subject's fasting blood ammonia level is greater than or equal to 1.0 ULN, then the subject's relative incidence of HAC per year may be about 1.071. In certain embodiments, the subject may have been previously treated with NaPBA.

According to other disclosures, if the subject has been previously treated with GPB, then the subject's overall relative incidence of HAC per year may be about 0.288. For example, this means that if 100 subjects that were treated with GPB were followed for one year, the expected number of HACs would be about 29. In certain disclosures, if the subject has been previously treated with GPB and the subject's fasting blood ammonia level is less than 0.5 ULN, then the subject's relative incidence of HAC per year may be about 0.135. In certain disclosures, if the subject has been previously treated with a dosage of GPB and the subject's fasting blood ammonia level is between 0.5 to less than 1.0 ULN, then the subject's relative incidence of HAC per year may be about 0.150. In certain disclosures, if the subject has been previously treated with a dosage of GPB and the subject's fasting blood ammonia level is greater than or equal to 1.0 ULN, then the subject's relative incidence of HAC per year may be about 0.711. In certain embodiments, the subject has been previously treated with GPB for an amount of time to maintain a steady state level of the drug in the subject. For example, the subject has been treated with a GPB for at least 3, 4, or 5 days. In some embodiments, the subject has been treated with GPB for about one year.

Additionally, age can also be useful for predicting a subject's relative incidence rate of HAC. Insome disclosures, if the subject has been treated with a nitrogen scavenging drug and the subject is less than eighteen years of age, then the incidence rate of HAC may be 2.6 times higher for the subject than for a subject that is eighteen years of age or older. In certain embodiments, the subject has been treated with GPB for an amount of time to maintain a steady state level of the drug in the subject. For example, the subject has been treated with a GPB for at least 3, 4, or 5 days.
In certain embodiments, the subject has been treated with GPB
for about one year.

As shown in Example 4 below, ammonia exposure can be useful for predicting the duration of an HAC. For example, in some disclosures, if the subject has a fasting ammonia level less than 1.0 ULN, the subject may have a higher likelihood of experiencing a short HAC duration compared to subjects with a fasting ammonia level greater than or equal to 1.0 ULN. In certain embodiments, the subject has been treated with GPB for an amount of time to maintain a steady state level of the drug in the subject. For example, the subject has been treated with a GPB for at least3, 4, or 5 days. In some embodiments, the subject has been treated with GPB for about one year. In certain disclosures, a short HAC duration is less than two days.

As shown in Example 5 below, age can be useful for predicting a subject's risk of having an HAC. For example, there was a significant association between fasting ammonia ULN categories and time from baseline to first HAC for patients who were at least 6 years of age. The time to first HAC was significantly shorter in patients with a fasting ammonia level greater than or equal to 1.0 ULN compared to those patients with a fasting ammonia level less than 0.5 ULN. In some disclosures, if a subject's fasting ammonia level is between 0.5 and less than 1.0 ULN and the subject is 6 years or older, the subject may have about a three times higher rate of HAC compared to a subject with a fasting ammonia level less than 0.5 ULN. In certain disclosures, if a subject's fasting ammonia level is equal to or greater than 1.0 ULN and the subject is 6 years or older, the subject may have about a twenty times higher rate of HAC compared to a subject with a fasting ammonia level less than 0.5 ULN. In certain disclosures, the HAC is a first HAC. In certain embodiments, the subject has been treated with GPB for an amount of time to maintain a steady state level of the drug in the subject. For example, the subject has been treated with a GPB for at least 3, 4, or 5 days. In some embodiments, the subject has been treated with GPB for about one year.

Example 6 below demonstrates the relative risk of experiencing an HAC based on incremental increases in blood ammonia. Total ammonia exposure expressed as 12 month time normalized under the curve (TNAUC₁₂ₘₒₙₜₕₛ) was significantly lower in patients who experienced an HAC compared to those who did not. In certain disclosures, a fasting ammonia level of a subject is taken and compared with a fasting blood ammonia level taken when the subject did not experience an HAC. In certain disclosures, the subject's fasting ammonia level may be compared to a control level of blood ammonia level from other subjects who did not experience an HAC. In certain disclosures, if a subject's fasting blood ammonia level is 5 µmol/L greater than the baseline ammonia level, the subject is administered a nitrogen scavenging drug.

In certain disclosures, if a subject's fasting blood ammonia level is 5 µmol/L greater than the baseline ammonia level, the risk of an HAC is about 23%. In certain disclosures, if a subject's fasting blood ammonia level is 10 µmol/L greater than the baseline ammonia level, the risk of an HAC is about 50%. In certain disclosures, if a subject's fasting blood ammonia level is 25 µmol/L greater than the baseline ammonia level, the risk of an HAC is about 270%. In certain disclosures, the subject is administered a nitrogen scavenging drug if the risk of HAC is greater than about 23%. In certain disclosures, the subject has been treated with GPB, for an amount of time to maintain a steady state level of the drug in the subject. For example, the subject has been treated with a GPB for at least 3, 4, or 5 days. In some disclosures, the subject has been treated with GPB for about one year.

Example 8 shows that glutamine levels were generally higher in patients in the higher baseline ammonia ULN groups and had decreased in the patients with the highest baseline glutamine at baseline by three months of treatment. Additionally, there was a statistically significant but comparatively weak correlation between baseline ammonia and glutamine levels at the time of enrollment as well as between fasting glutamine levels and daily ammonia exposure on GPB or NaPBA. An analysis of the relationship between glutamine and ammonia categories with respect to ULN categories showed that the overall correlation appeared to be driven by patients whose a baseline ammonia exceeded 1.0 ULN. Further, patients with OTC and CPS showed a stronger correlation between glutamine and ammonia.

Moreover, glutamine levels were slightly higher at baseline in patients who experienced an HAC compared to patients who did not experience an HAC during the study. In certain embodiments, if a subject has been previously treated with a nitrogen scavenging drug and has a glutamine level between about 649 µmol/L to 808 µmol/L, then the subject has a greater likelihood of risk of HAC compared to subjects with a glutamine level less than about 649 µmol/L. In certain embodiments, the risk of HAC may be about a 2.5 times higher rate of risk. According to certain embodiments, if a subject has been previously treated with a nitrogen scavenging drug and has a glutamine level greater than or equal to about 809 µmol/L, then the subject has a greater likelihood of risk of HAC compared to subjects with a glutamine level less than about 649 µmol/L. In certain embodiments, the risk of HAC may be about a 2.8 times higher rate of risk. In certain embodiments, the subject has been treated with GPB, for an amount of time to maintain a steady state level of the drug in the subject. For example, the subject has been treated with a GPB for at least 3, 4, or 5 days. In some embodiments, the subject has been treated with GPB for about one year. In certain embodiments, the glutamine upper limit normal values are: for patients 4 months to 2 years of age (709 µmol/L), 2 to 10 years of age (709 µmol/L), 10 to 18 years of age (740 µmol/L), older than 18 years of age (721 µmol/L) (Blau 2008).

The ULN for blood ammonia typically represents the highest level in the range of normal values, which may be influenced by a variety of factors such as the assay method, types of regents, standard reference samples used, and specifications and calibration of equipment used to perform the measurement. In certain embodiments of the methods disclosed herein, the ULN for blood ammonia is determined for a subject individually. In other embodiments, the ULN for blood ammonia may be based on measurements obtained across a set of subjects (e.g., healthy subjects or subjects with UCD). In certain embodiments, the ULN for blood ammonia may represent a standard reference value disclosed in the art, such as a mean ULN developed across a particular subset of subjects. In other embodiments, the ULN for blood ammonia may represent a standard measurement that has been developed by a particular entity that performs blood draws and/or blood evaluations, such as a particular clinical laboratory. In certain embodiments, the ULN is a standard reference value utilized by the same entity that measures the fasting blood ammonia level. In these embodiments, one skilled in the art will recognize that the units of ammonia measurement may also vary from lab to lab (e.g., µg/mL or µmoI/L), emphasizing the importance of interpreting the subject's ammonia levels relative to the ULN at the laboratory in which the measurement was performed. In certain embodiments, the ULN for blood ammonia may be about 12 to 70 µmol/L. In certain of these embodiments, the ULN for blood ammonia may be about 11 to 64 µmol/L, 20 to 50 µmol/L, 30 to 40 µmol/L, 32 to 38 µmol/L, or 34 to 36 µmol/L, and in certain of these embodiments the ULN for blood ammonia is about 35 µmol/L. In certain embodiments, the ULN for blood ammonia may be about 20 to 120 µg/dL. In certain of these embodiments, the ULN for blood ammonia may be about 50 to 65 µg/dL, 55 to 63 µg/dL, or 57 to 61 µg/dL, and in certain of these embodiments the ULN for blood ammonia is about 59 µg/dL.

A nitrogen scavenging drug as used herein refers to any drug that decreases blood nitrogen and/or ammonia levels. In certain embodiments, a nitrogen scavenging drug may remove nitrogen in the form of PAGN, and in certain of these embodiments the nitrogen scavenging drug may be an orally administrable drug that contains or is metabolized to PAA. For example, a nitrogen scavenging drug may be a PAA prodrug such as PBA or GPB, a pharmaceutically acceptable salt of PBA such as NaPBA, or a pharmaceutically acceptable ester, acid, or derivative of a PAA prodrug. In other embodiments, a nitrogen scavenging drug may remove nitrogen via hippuric acid. In certain of these embodiments, a nitrogen scavenging drug may be benzoic acid, a pharmaceutically acceptable salt of benzoic acid such as sodium benzoate, or a pharmaceutically acceptable ester, acid, or derivative of benzoic acid. The nitrogen scavenging drug used in the invention is GPB.

Increasing the dosage of a nitrogen scavenging drug may refer to increasing the amount of drug per administration (e.g., an increase from a 3 mL dosage to a 6 mL dosage), increasing the number of administrations of the drug (e.g., an increase from once-a-day dosing to twice- or three-times-a-day), or any combination thereof.

In certain embodiments, a subject that has previously been administered a nitrogen scavenging drug has been administered the drug for a duration of time sufficient to reach steady
state. Similarly, in those methods where fasting blood ammonia level is measured following a first, second, third, or subsequent dosage of nitrogen scavenging drug, the measurement may be carried out after the drug has had sufficient time to reach steady state at that dosage. For example, the subject may have been administered the drug over a period of about 2 to 7 days, 1 week to 2 weeks, 2 weeks to 4 weeks, 4 weeks to 8 weeks, 8 weeks to 16 weeks, or longer than 16 weeks.

In certain embodiments of the methods disclosed herein, the fasting period for obtaining a fasting blood ammonia level is overnight. In certain embodiments, the fasting period is 4 hours or more, 5 hours or more, 6 hours or more, 7 hours or more, 8 hours or more, 9 hours or more, 10 hours or more, 11 hours or more, or 12 hours or more, and in certain embodiments the fasting period is 4 to 8 hours, 6 to 8 hours, or 8 to 12 hours. During the fasting period, the subject preferably does not ingest any food. In certain embodiments, the subject may also refrain from ingesting certain non-food substances during the fasting period. For example, in certain embodiments the subject does not ingest any supplements and/or nitrogen scavenging drugs during the fasting period. In certain of these embodiments, the subject may nonetheless ingest one or more drugs other than nitrogen scavenging drugs during the fasting period. In certain embodiments, the subject does not ingest any high calorie liquids during the fasting period. In certain of these embodiments, the subject does not ingest any liquids other than water during the fasting period. In other embodiments, the subject may ingest small amounts of low calorie beverages, such as tea, coffee, or diluted juices.

In certain embodiments of the methods disclosed herein, blood samples used for measuring fasting blood ammonia levels and/or ULN blood ammonias are venous blood samples. In certain embodiments, a blood sample is a plasma blood sample. Any methods known in the art may be used to obtain a plasma blood sample. For example, blood from a subject may be drawn into a tube containing heparin or ethylenediaminetetraacetic acid (EDTA). In certain embodiments, the sample can be placed on ice and centrifuged to obtain plasma within 15 minutes of collection, stored at 2 to 8°C (36 to 46°F) and analyzed within 3 hours of collection. In other embodiments, the blood plasma sample is snap frozen, stored at ≤-18°C (≤0°F) and analyzed at a later time. For example, the sample may be analyzed at 0 to 12 hours, 12 to 24 hours, 24 to 48, 48 to 96 hours after freezing, or within any other timeframe over which the sample has demonstrated stability. In certain embodiments, blood samples are taken in a laboratory or hospital setting. In certain embodiments, a single fasting blood sample is used to measure fasting blood ammonia level. However, in other embodiments, multiple fasting blood samples may be obtained. In certain embodiments, a subject's blood ammonia level may be monitored throughout the day. Further, in certain embodiments, the methods disclosed herein comprise an additional step of obtaining one or more blood samples from a subject prior to or after measuring fasting blood ammonia level.

In certain embodiments, a blood sample is analyzed immediately after collection. In other embodiments, the blood sample is stored for some period between collection and analysis. In these embodiments, the sample may be stored for less than 1 hour, 1 hour to 6 hours, 1 hour to 12 hours, 1 hour to 24 hours, or 1 hour to 48 hours. In certain of these embodiments, the blood sample is stored at a temperature between 0 to 15°C, such as 2 to 8°C. In other embodiments, the blood sample is stored below 0°C or below -18°C.

Measurement of ammonia levels in a fasting blood sample may be carried out using any technique known in the art. For example, ammonia levels may be measured using a colorimetric reaction or an enzymatic reaction. In certain embodiments, a colorimetric reaction may involve the use of bromophenol blue as an ammonia indicator. In these embodiments, ammonia may react with bromophenol blue to yield a blue dye. In certain embodiments, an enzymatic reaction may involve glutamate dehydrogenase catalyzing the reductive amination of 2-oxoglutarate with NH⁴⁺ and NADPH to form glutamate and NADP⁺. The formation of NADP⁺ formed is directly proportional to the amount of ammonia present in the blood sample. Therefore, the concentration of ammonia is measured based on a decrease in absorbance.

One skilled in the art will recognize that a variety of other factors may be taken into consideration when determining the effective dosage of GPB. For example, factors such as diet (e.g., protein intake) and endogenous waste nitrogen removal capacity (e.g., urea synthesis capacity) may be considered.

One of ordinary skill in the art will recognize that the various embodiments described herein can be combined. For example, steps from the various methods of treatment disclosed herein may be combined in order to achieve a satisfactory or improved level of treatment.

The following examples are provided to better illustrate the claimed invention. The scope of the invention is defined in the appended claims.

### Examples

### Example 1: Relationship between fasting ammonia levels and HAC during long-term (1 year) treatment of UCD patients.

Data for 100 UCD subjects from three clinical trials (HPN-100-005, HPN-100-007, and HPN-100-012) were analyzed to evaluate the correlation between fasting ammonia levels and HAC (Table 1). Patients were treated for 12 months with GPB during the study. There was an almost equal split in the number of subjects who were adults (51%) and children (49%), defined as age <18 years at the time of enrollment. In this population, 30 patients had experienced a total of 54 HACs in the 12 months prior to the study and 19 patients experienced a total of 27 HACs during the study (Table 1). Baseline ammonia was defined as the subject's fasting ammonia level taken either at month zero or at the screening visit depending on which study the subject was enrolled in and whether the subject was enrolled in previous studies.

**Table 1. Descriptive statistics of the study population.**

| | **STUDY** | | | |
|---|---|---|---|---|
| | **TOTAL** | **005** | **007** | **012** |
| **N** | 100 | 17 | 60 | 23 |

| # **of HA events** | | | | |
|---|---|---|---|---|
| Pre-study | 54 | 8 | 17 | 29 |
| Study | 27 | 3 | 12 | 12 |

| **N with ≥1 HA events** | | | | |
|---|---|---|---|---|
| Pre-study | 30 | 5 | 10 | 15 |
| Study | 19 | 3 | 9 | 7 |

| **Age at baseline** | | | | |
|---|---|---|---|---|
| Mean in years | 19.6 | 10.0 | 28.8 | 2.8 |
| (SD) | (15.9) | (3.5) | (13.9) | (1.7) |

| **Dichotomous age (%)** | | | | |
|---|---|---|---|---|
| Pediatric (<18 years) | 49.0 | 100.0 | 15.0 | 100.0 |
| Adult (≥18 years) | 51.0 | 0.0 | 85.0 | 0.0 |

| **Baseline ammonia (µmol/L)** | | | | |
|---|---|---|---|---|
| Mean | 28.8 | 27.7 | 27.5 | 32.8 |
| (SD) | (19.9) | (12.4) | (19.6) | (24.9) |
| 25th percentile | 11.0 | 14.0 | 10.0 | 11.0 |
| 50th percentile | 28.9 | 29.0 | 26.5 | 25.0 |
| 75th percentile | 37.5 | 37.0 | 36.7 | 51.0 |
| 0 to <0.5 normal limit | 39.0% | 29.4% | 40.0% | 43.5% |
| 0.5 to <1.0 normal limit | 34.0% | 58.8% | 30.0% | 26.1% |
| ≥1 normal limit | 27.0% | 11.8% | 30.0% | 30.4% |

| **Gender (%)** | | | | |
|---|---|---|---|---|
| Male | 33.0 | 17.7 | 31.7 | 47.8 |
| Female | 67.0 | 82.3 | 68.3 | 52.2 |

| **Race (%)** | | | | |
|---|---|---|---|---|
| White | 81.0 | 82.4 | 80.0 | 82.6 |
| Non-white | 19.0 | 17.6 | 20.0 | 17.4 |

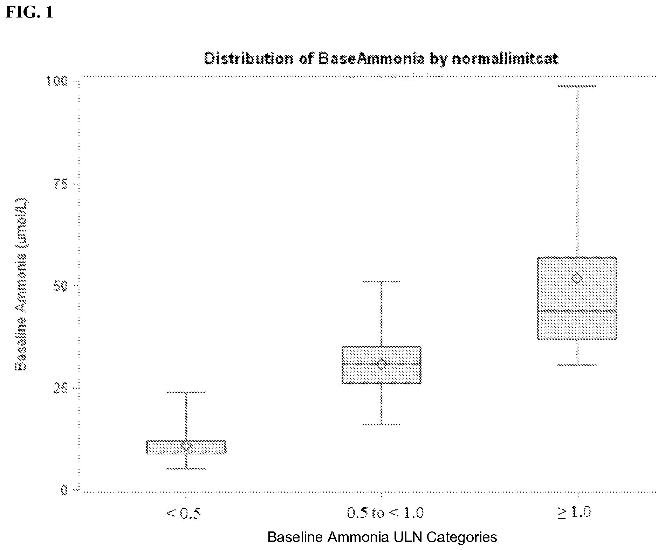

Subjects were divided into three groups based on their fasting ammonia levels: 0 to less than 0.5 (0 to <0.5 ULN) times the upper limit normal (ULN) (39 subjects), 0.5 to less than 1.0 (0.5 to <1.0 ULN) times ULN (34 subjects), and 1.0 times ULN or greater (≥1.0 ULN) (27 subjects). To characterize the distribution of baseline ammonia for the upper limit normal categories of baseline ammonia, boxplots of ammonia for upper limit normal categories were developed as shown in FIG. 1. There was very little overlap in raw baseline ammonia values across baseline ammonia upper limit normal categories. The descriptive statistics of baseline ammonia categories are provided in Table 2. An increasing trend was observed across the ULN baseline ammonia categories in mean, median and range. No substantial differences between categories were observed at baseline among the different groups in terms of age, gender or UCD subtype, although there was a slightly higher proportion of patients aged 6 to 11 years in the 0.5 to <1.0 ULN group than the other two groups. Additionally, glutamine levels increased with increasing baseline ammonia. Seven patients were less than 2 years old, with a similar proportion of these patients in each of the baseline ammonia categories. Patients with ornithine transcabamylase (OTC) deficiency, the most common UCD subtype and one frequently associated with milder disease, were also evenly distributed among the subgroups.

**Table 2: Descriptive statistics of baseline ammonia (µmol/L) stratified by the baseline ammonia categorized in relation to ULN.**

| **Variable** | **Baseline Ammonia Category** | | |
|---|---|---|---|
| | **< 0.5 ULN** | **0.5 to < 1.0 ULN** | **≥ 1.0 ULN** |
| | **(N = 39)** | **(N = 34)** | **(N = 27)** |
| **Age** | | | |
| Mean in years | 22.9 | 17.4 | 18.6 |
| (SD) | (17.7) | (15.0) | (12.8) |

| **Number of patients** | | | |
|---|---|---|---|
| Adult (≥ 18 years) | 22 | 14 | 15 |
| (%) | (56.4) | (41.2) | (55.6) |
| Pediatric (<18 years) | 17 | 20 | 12 |
| (%) | (43.6) | (58.8) | (44.4) |
| <2 years | 3 | 2 | 2 |
| (%) | (7.7) | (5.9) | (7.4) |
| 2 to 5 years | 7 | 4 | 5 |
| (%) | (17.9) | (11.8) | (18.5) |
| 6 to 11 years | 5 | 10 | 2 |
| (%) | (12.8) | (29.4) | (7.4) |
| 12 to 17 years | 2 | 4 | 3 |
| (%) | (5.1) | (11.8) | (11.1) |

| **Gender** | | | |
|---|---|---|---|
| Male | 15 | 9 | 9 |
| (%) | (38.5) | (26.5) | (33.3) |
| Female | 24 | 25 | 18 |
| (%) | (61.5) | (73.5) | (66.7) |

| **Fasting Baseline Ammonia (µmol/L)** | | | |
|---|---|---|---|
| Mean | 11 | 30.8 | 51.9 |
| (SD) | (3.7) | (7.7) | (19.5) |

| **Glutamine at Baseline** | | | |
|---|---|---|---|
| Mean | 694 | 710 | 857 |
| (SD) | (199.0) | (183.3) | (306.0) |

| **UCD Subtype** | | | |
|---|---|---|---|
| ARG | 1 | 0 | 1 |
| (%) | (2.6) | | (3.7) |
| ASL | 7 | 5 | 1 |
| (%) | (18.0) | (14.7) | (3.7) |
| ASS | 4 | 3 | 5 |
| (%) | (10.3) | (8.8) | (18.5) |
| CPS | 1 | 0 | 0 |
| (%) | (2.6) | | |
| HHH | 2 | 1 | 0 |
| (%) | (5.1) | (2.9) | |
| OTC | 24 | 25 | 20 |
| (%) | (61.5) | (73.5) | (74.1) |

Next, the subjects were analyzed according to whether or not they had experienced an HAC prior to enrollment and those that experienced an HAC during the clinical trials. Nineteen patients (19.0%) experienced a total of 27 HACs during study treatment. The proportion of patients who experienced an HAC during the study increased with increasing baseline ammonia. The proportion of patients experiencing an HAC was also higher for pediatric than adult patients and for male patients than female patients (Table 3).

The proportion of patients who experienced an HAC on NaPBA treatment prior to enrollment (30.0%), based on retrospectively collected data, was higher than the proportion of patients who experienced an HAC during the study. A total of 54 HACs were reported during the pre-study period. Similar to the on-study data, the pre-study data showed that the proportion of patients with HAC increased with increasing baseline ammonia and that HAC were experienced by a higher proportion of pediatric than adult patients and a higher proportion of male than female patients (Table 3). The most common UCD subtype in this study was ornithine transcabamylase (OTC) deficiency (n = 69). The proportion of patients who experienced an HAC during the study was similar for those with OTC deficiency (18.8%) and those with non-OTC deficiencies (19.4%).

**Table 3: Proportion of patients who experienced an HAC.**

| | **Pre-Study** | **During Study** |
|---|---|---|
| **Number of patients with HAC** | 30 | 19 |
| **(%)** | (30.0) | (19.0) |
| **Total no. of HAC** | 54 | 27 |

| **Age (% of patients)** | | |
|---|---|---|
| Pediatric (< 18 years) | 42.9 | 24.5 |
| Adult (≥ 18 years) | 17.7 | 13.7 |

| **Baseline Ammonia (µmol/L)** | | |
|---|---|---|
| 0 to <0.5 ULN | 23.1 | 10.3 |
| 0.5 to <1.0 ULN | 26.5 | 14.7 |
| ≥ 1.0 ULN | 44.4 | 37.0 |

| **Gender (% of patients)** | | |
|---|---|---|
| Male | 42.4 | 24.2 |
| Female | 23.9 | 16.4 |

| **Race (% of patients)** | | |
|---|---|---|
| White | 28.4 | 18.5 |
| Non-white | 36.8 | 21.1 |

Next, the subjects were analyzed according to whether or not they had experienced an HAC during the clinical trials compared with those that did not experience an HAC. The characteristics of the HAC and non-HAC groups are summarized in Table 4.

**Table 4: Descriptive statistics of subjects who experienced an HAC and those that did not experience an HAC during the study period.**

| | | **HAC** | **No HAC** |
|---|---|---|---|
| | | **(N=19)** | **(N=81)** |
| **Overall %** | | 19.0 | 81.0 |
| **Mean age in years** | | 12.9 | 21.2 |
| **(SD)** | | (11.7) | (16.3) |
| **Age ranges (%)** | **<6 years** | 30.4 | 69.6 |
| | **6-11 years** | 23.5 | 76.5 |
| | **12-17 years** | 11.1 | 88.9 |
| | **≥18 years** | 13.7 | 86.3 |
| **Dichotomous age (%)** | **Pediatric: <18 years** | 24.5 | 75.5 |
| | **Adult: >vears** | 13.7 | 86.3 |
| **Mean baseline ammonia (µmol/L)** | | 41.8 µmol/L | 25.7 µmol/L |
| **(SD)** | | (23.7) | (17.8) |
| **Baseline ammonia (vs. ULN)** | **0 - <0.5 ULN** | 10.3 | 89.7 |
| | **0.5** - **<1.0 ULN** | 14.7 | 85.3 |
| | **≥1 ULN** | 37.0 | 63.0 |
| **Gender** | **Male** | 24.2 | 75.8 |
| **(%)** | **Female** | 16.4 | 83.6 |
| **Race** | **White** | 18.5 | 81.5 |
| **(%)** | **Non-white** | 21.1 | 78.9 |

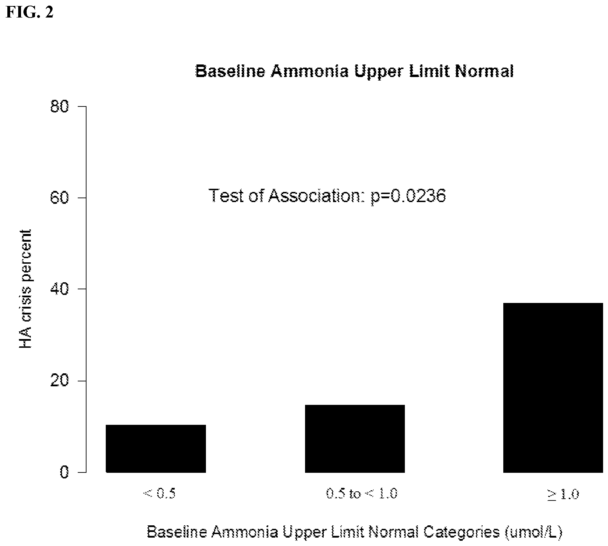

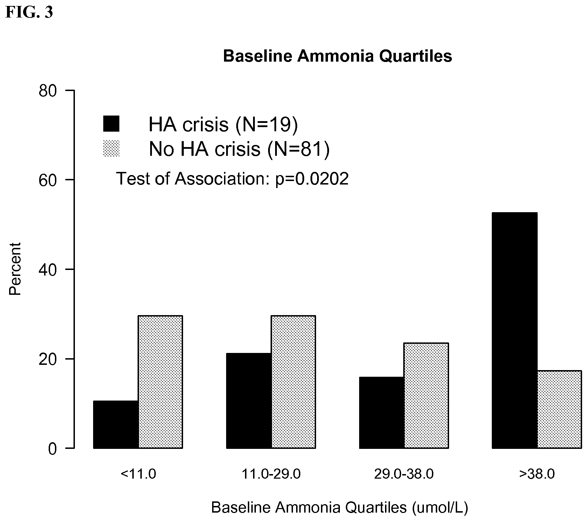

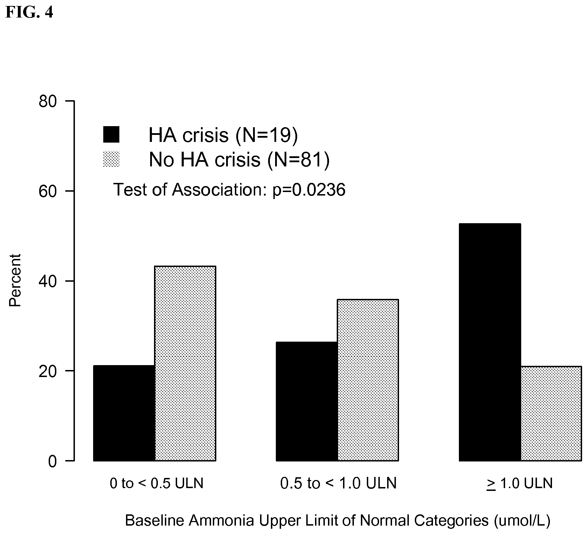

Nineteen of the subjects had experienced an HAC during the clinical trials, while 81 had not. The mean age of subjects who experienced an HAC was lower than the subjects who did not have an HAC (12.9 years versus 21.5 years). The mean baseline ammonia was about two times higher for subjects who experienced an HAC verses those who did not. Additionally, the percentage of subjects who experienced an HAC during the study increased as baseline ammonia levels increased, with subjects experiencing HACs exhibiting baseline ammonia levels approximately 1.5 times higher than subjects who did not experience HAC (10%, 15%, and 37% for patients with baseline ammonia < 0.5 ULN, 0.5 to <1.0 ULN, ≥ 1.0 ULN, respectively). The occurrence of HACs for subjects with fasting ammonia levels of <0.5 ULN, 0.5 to <1 ULN, and equal to or greater than 1.0 ULN is summarized in FIGs. 2, 3, and 4. The association between ULN categories and HAC was significant so that the percentage of patients who experienced an HAC was lowest in those with a baseline ammonia of <0.5 ULN and increased in a step-wise fashion to those with <1.0 ULN and was highest in patients with the ≥ 1.0 ULN ammonia value at baseline (p=0.024). Thus, fasting ammonia categories provide a useful predictor of HAC likelihood, and subjects falling in the fasting ammonia category of <0.5 ULN have a significantly lower likelihood of experiencing an HAC.

### Example 2: Relationship between fasting ammonia levels and time to first HAC.

Subjects were monitored over 400 days and factors that may influence a subject's time to first HAC were analyzed. The probability of experiencing an HAC for the whole population and those falling in each ULN category is plotted using the Kaplan Meier (KM) (Kaplan 1958). The y-axis of the KM plot is the survival probability (HAC-free survival) and the x-axis is time in days. The study population begins at 100% HAC free and the KM plot describes the probability of not having an HAC over time and "+" denotes a censored value. A censored value denotes that the subject is no longer being followed in the study. Log rank tests were computed to compare the survival distributions between the covariant values (Peto 1972).

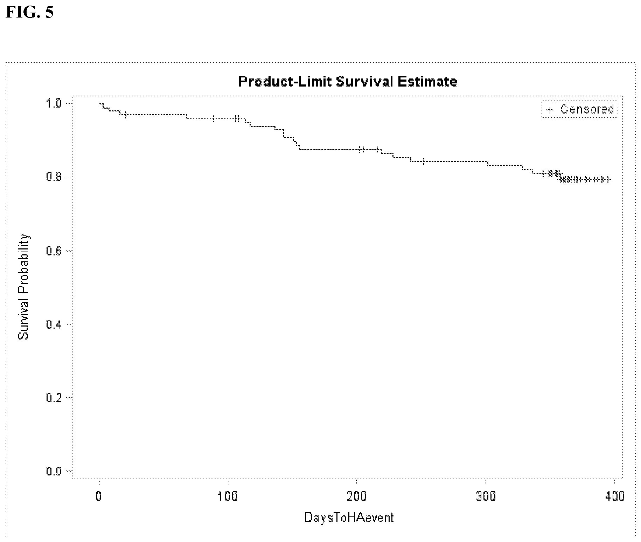

Crisis-free survival for the entire study population is depicted in FIG. 5. As shown, after about one year of follow-up, 80% of subjects had not experienced an HAC during treatment with GPB (FIG. 5). The KM plot was relatively flat until about day 150 when the curve experienced a significant drop. This suggested that a good number of subjects experienced an HAC around 150 days after which the KM plot begins to level off.

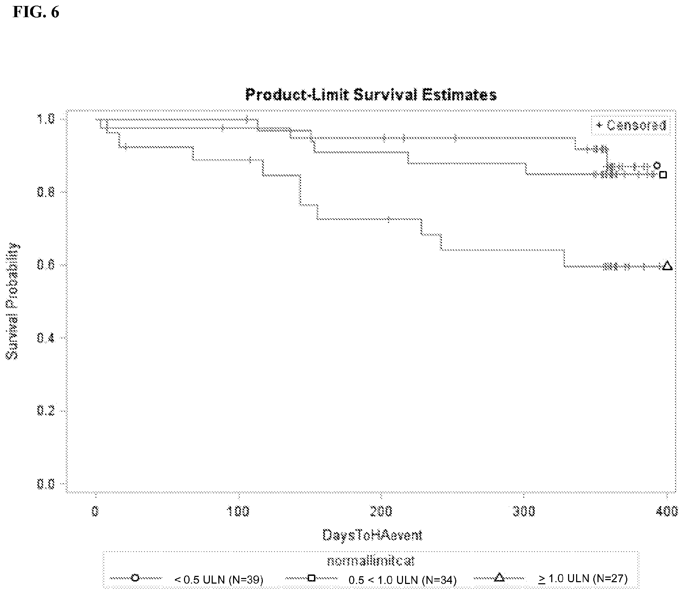

Next, subjects diagnosed with UCD were divided into three groups based on their fasting ammonia levels: < 0.5 ULN, 0.5 to <1.0 ULN, ≥ 1.0 ULN. Results are set forth in FIG. 6. Subjects with higher baseline ammonia levels relative to ULN were found to be at greater risk of HAC compared to patients in lower ULN categories, and exhibited a lower survival probability. There were statistically significant associations between the time to first HAC and baseline ammonia quartiles (p=0.0088) and between time to first HAC and baseline ammonia by ULN category (p=0.0077). Patients with higher baseline ammonia by quartiles or ULN category were at greater risk of HAC than patients with lower baseline ammonia (FIG. 6).

In order to quantify the relative risk of time to first HAC, univariable Cox proportional hazard model regressions were generated. These results are set forth in Table 5.

**Table 5: Univariable Cox regression results for time to first HAC.**

| **Risk factor** | | **N** | **Hazard ratio** | **p-value** |
|---|---|---|---|---|
| **Age in years** | **Pediatric (<18 years)** | 49 | 1.84 | 0.200 |
| | **Adult (≥18 years)** | 51 | Ref | |
| **Baseline ammonia (vs. ULN)** | **0 - <0.5 ULN** | 39 | Ref | Ref |
| | **0.5 - <1.0 ULN** | 34 | 1.43 | 0.598 |
| | **≥1.0 ULN** | 27 | 4.51 | 0.011 |
| **Gender** | **Male** | 33 | 1.58 | 0.327 |
| **(%)** | **Female** | 67 | Ref | Ref |
| **Race** | **White** | 81 | Ref | Ref |
| **(%)** | **Non-white** | 19 | 1.19 | 0.760 |

The data suggested that the risk of HAC was about 4.5 times higher in subjects with baseline ammonia levels of 1.0 times ULN or greater versus subjects with baseline ammonia levels at <0.5 times ULN. This result was statistically significant (p=0.011).

A multivariable cox regression model for each of the risk factors in Table 6 was generated to account for potential cofounders. The hazard ratios are presented in Table 6.

**Table 6: Multivariable Cox regression results for time to first HAC during the study.**

| **Risk Factor** | **N** | **Hazard Ratio** | **p-value** |
|---|---|---|---|
| **Age in years** | | | |
| **Pediatric: <18** | 49 | 2.60 | 0.062 |
| **Adult: ≥18** | 51 | Ref | |

| **Baseline Ammonia (µmol/L)** | | | |
|---|---|---|---|
| **0 - <0.5 ULN** | 39 | Ref | Ref |
| **0.5** - **<1.0 ULN** | 34 | 1.49 | 0.562 |
| **≥1.0 ULN** | 27 | 5.67 | 0.005 |

| **Gender (%)** | | | |
|---|---|---|---|
| **Male** | 33 | 2.05 | 0.153 |
| **Female** | 67 | Ref | Ref |

| **Race (%)** | | | |
|---|---|---|---|
| **White** | 81 | Ref | Ref |
| **Non-white** | 19 | 0.81 | 0.713 |

After adjusting for age, gender and race, there was still a significant effect of baseline ammonia on the risk of HAC. Specifically, subjects with a baseline ammonia of one or more of their upper limit normal value have about 5.7 times the risk of having an HAC compared to subjects with <0.5 ULN baseline ammonia values (p=0.005). This further supports earlier findings showing that determining baseline ammonia levels as a function of ULN is a useful diagnostic tool.

### Example 3: Relationship between fasting ammonia levels and cumulative HACs.

Some subjects experienced more than one HAC during follow-up treatment. Thus, the rate of HAC over 12 months was examined in relation to baseline characteristics. The majority of subjects had at least 12 months of follow up information as the median time a subject was followed during the study was about one year (i.e., 361.0 days). To estimate HAC rates and compare the differences between covariates of interest, a univariable negative binomial regression model was generated. These results are set forth in Table 7.

**Table 7: Univariable Negative Binomial regression results showing relative incidence for HAC in relation to potential risk factors.**

| **Risk factor** | | **Pre-enrollment** | | | **During study** | | |
|---|---|---|---|---|---|---|---|
| | | **12-month incidence** | **HAC incidence rate ratio (IRR)** | **p-value** | **12-month incidence** | **HAC incidence rate ratio (IRR)** | **p-value** |
| **Overall** | | 0.581 | | | 0.288 | | |
| **Age in years** | **Pediatric (<18 years)** | 0.338 | Ref | | 0.209 | Ref | |
| | **Adult (≥18 years)** | 0.832 | 2.46 | 0.346 | 0.371 | 1.78 | 0.2539 |
| **Baseline ammonia (vs. ULN)** | **0 - <0.5 ULN** | 0.433 | Ref | | 0.135 | Ref | |
| | **0.5 - <1.0 ULN** | 0.389 | 0.90 | 0.8161 | 0.150 | 1.11 | 0.8674 |
| | **≥1.0 ULN** | 1.071 | 2.47 | 0.0457 | 0.711 | 5.28 | 0.0057 |
| **Gender (%)** | **Male** | 0.516 | Ref | | 0.275 | Ref | |
| | **Female** | 0.712 | 1.38 | 0.3949 | 0.314 | 1.14 | 0.7832 |
| **Race (%)** | **White** | 0.564 | Ref | | 0.296 | Ref | |
| | **Non-white** | 0.660 | 1.17 | 0.7246 | 0.248 | 0.838 | 0.7456 |

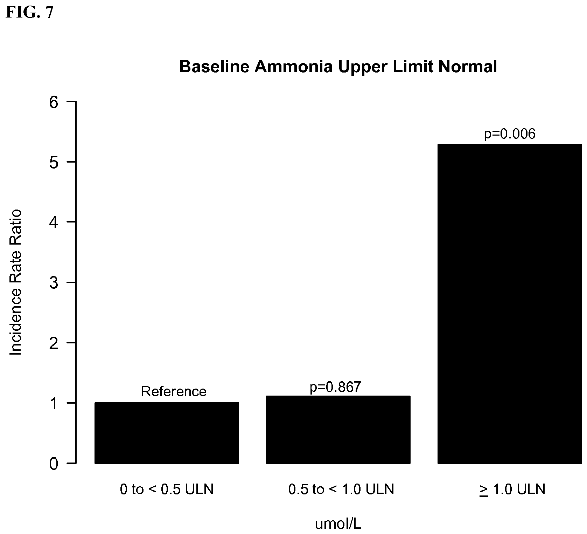

The pre-enrollment HAC incidence rate was 0.581, suggesting that if 100 subjects were followed for a year, the expected number of HACs would be around 58. The study HAC incidence rate was 0.288, i.e., roughly 50% lower than the pre-enrollment rate. The risk of HAC was about five times higher for subjects with baseline ammonia levels of 1.0 ULN or greater versus subjects with baseline ammonia levels at <0.5 ULN. The incidence rate ratios for different baseline ammonia categories are set forth in FIG. 7. These results further illustrate the large gap in outcomes between subjects in the first two categories (<0.5 ULN and 0.5- < 1.0 ULN) and subjects in the third category (equal to or greater than 1.0 ULN).

To adjust for potential cofounders, a multivariable Negative Binomial regression of HAC rate on baseline ammonia ULN categories and subject demographics was developed as presented in Table 8.

**Table 8: Multivariable Negative Binomial regression results for rates of HAC during the study.**

| **Risk Factor** | **Incidence Rate Ratio** | **p-value** |
|---|---|---|
| **Age in years** | | |
| **Adult: ≥18** | ref | |
| **Pediatric: <18** | 2.62 | 0.0543 |

| **Baseline Ammonia (µmol/L)** | | |
|---|---|---|
| **0 - <0.5 ULN** | ref | |
| **0.5 - <1.0 ULN** | 1.11 | 0.8648 |
| **≥1.0 ULN** | 7.38 | 0.0004 |

| **Gender (%)** | | |
|---|---|---|
| **Female** | ref | |
| **Male** | 1.79 | 0.2229 |

| **Race (%)** | | |
|---|---|---|
| **White** | ref | |
| **Non-white** | 0.53 | 0.2444 |

After adjusting for age, gender and race, a significant difference in HAC incidence was observed between baseline ammonia categories (p= 0.0004). Age showed a marginally significant difference in HAC incidence after controlling for baseline ammonia, gender and race. Notably, pediatric subjects showed a higher incidence of HAC compared to adult subjects (p= 0.0543).

### Example 4: Relationship between ammonia exposure and duration of HAC.

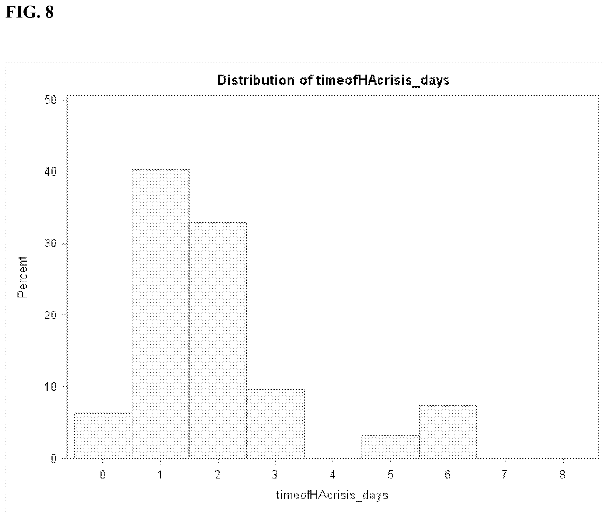

The relationship between ammonia exposure and duration of HAC was analyzed. Ammonia exposure was characterized three different ways: peak ammonia during crisis, total ammonia during crisis and ammonia at time of admission. The distribution of duration of HAC in subjects with HACs is shown in FIG. 8. The majority of the HACs typically lasted a day, and a steep drop off in the distribution was seen after 2 days. (Note: two subjects were excluded with 20 days of duration of HAC in this analyses). The descriptive statistics of the duration of HAC are presented in Table 9.

**Table 9: Descriptive statistics of the duration of HAC for study population.**

| | | **Study** | | |
|---|---|---|---|---|
| | **TOTAL** | **005** | **007** | **012** |
| **N** | **25** | **2** | **12** | **11** |
| **Duration of HA crisis** | | | | |
| **Mean (days)** | 1.71 | 1.27 | 1.95 | 1.53 |
| **Median (days)** | 1.00 | 1.27 | 1.36 | 0.92 |
| **< 1 days (%)** | 48.0 | 50.0 | 41.7 | 54.6 |
| **1 -1.99 days (%)** | 16.0 | 0.0 | 16.7 | 18.2 |
| **2 - 2.99 days (%)** | 28.0 | 50.0 | 33.3 | 18.2 |
| > **3 days (%)** | 8.0 | 0.0 | 8.3 | 9.1 |

Overall, about 48% of subjects experienced an HAC that lasted less than a day and about 64% of subjects had a duration of HAC that lasted less than 2 days.

To assess the association between duration of HAC and admission ammonia during HAC, a generalized estimation equation (GEE) logistic regression model was implemented with robust empirical standard errors that accounts for the clustering of HACs in some subjects (Hardin 2005). A linear mixed model could not be implemented because of convergence problems with such a small sample and outlier observations of admission ammonia. Instead, the duration of HAC was dichotomized to take on a value of 0 when the subject experienced an HAC that lasted 2 days or longer and 1 when the subject experienced an HA crisis of less than 2 days. The odds of experiencing an HAC of 2 days or less (Short HAC duration) on admission, peak and discharge ammonia as well as patient characteristics are modeled in Table 10.

**Table 10: GEE logistic regression model of the odds of having an HAC less than 2 days.**

| **Risk Factor** | **Odds Ratio** | **p-value** |
|---|---|---|
| **Admission Ammonia** | | |
| **100 µmol/L increase** | 1.46 | 0.269 |

| **Peak Ammonia** | | |
|---|---|---|
| **100 µmol/L increase** | 1.06 | 0.805 |

| **TNAUC Ammonia** | | |
|---|---|---|
| **100 µmol/L increase** | 1.00 | 0.958 |

| **Age in years** | | |
|---|---|---|
| **Pediatric: <18** | Ref | |
| **Adult: ≥18** | 1.74 | 0.565 |

| **Baseline Ammonia** | | |
|---|---|---|
| **0-38.0 µmol/L** | 3.06 | 0.279 |
| **38.0+ µmol/L** | Ref | |

| **Baseline Ammonia** | | |
|---|---|---|
| **0 - <1.0 ULN** | 3.06 | 0.279 |
| **≥1.0 ULN** | Ref | |

| **Gender (%)** | | |
|---|---|---|
| **Male** | 3.06 | 0.279 |
| **Female** | Ref | |

| **Race (%)** | | |
|---|---|---|
| **White** | DNC | DNC |
| **Non-white** | DNC | DNC |
| GEE: Generalized estimation equation | | |
| DNC: Did not converge | | |

As shown in Table 10, subjects with low baseline ammonia levels (0 to <1 ULN) had higher odds of experiencing short HAC duration compared to subjects with high baseline ammonia levels (≥1 ULN). These results support the data presented herein that ammonia levels influence HAC.

### Example 5: Relationship between different subsets of age and time to first HAC.

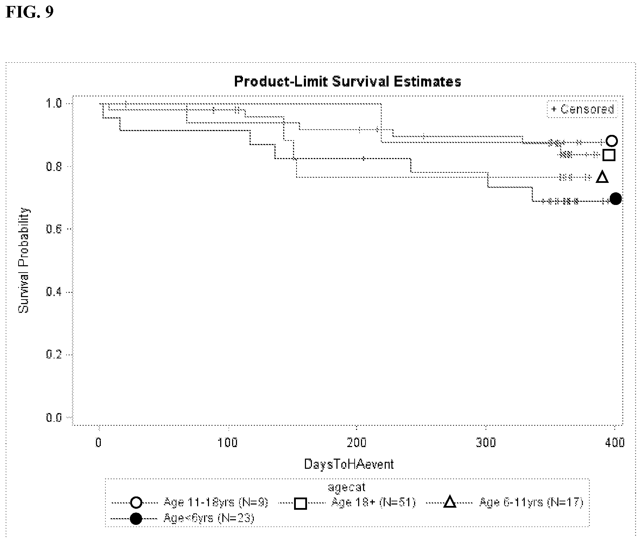

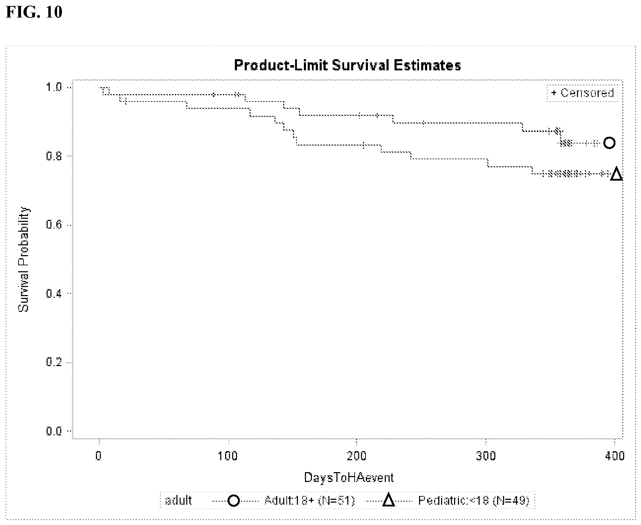

To analyze the relationship between age and HAC, characteristics of HAC were evaluated for subsets of subjects from different age groups. The effect of age on a subject's first HAC was investigated by generating KM plots that depict crisis-free survival in relation to four different subsets of age. A KM plot of time to first HAC for subsets of different ages (i.e., <6 years, 6-11 years, 11-18 years, and 18 years and older) showed that the younger patients seemed to have shorter time to the first HAC although no statistically significant association between these subsets of age and time to first HAC was observed as depicted in FIG. 9. In general, there was a separation in the survival curves after 150 days, with subjects ≤ 18 years of age experiencing a directionally higher risk of HAC as compared to adults. Next, a KM plot of time to first HAC by binary age subsets (adults: 18 years and older (N=51) vs pediatric: <18 years (N=49)) showed that children experienced a directionally higher risk of HAC. The KM plot is illustrated in FIG. 10.

To further investigate the relationship between age and time to first HAC, characteristics of HA crisis were evaluated for those patients in clinical studies HPN-100-005 and HPN-100-007 who were at least 6 years old at baseline (n = 77). The rationale for this analysis is that the fasting ammonia measurement is less reliable in very young children as infants and young children feed more frequently and were not in a true state of fasting like older patients at the time of ammonia measurement. Similar analyses were performed to analyze HAC risk in subjects six years of age or older. The characteristics of this subpopulation are set forth in Table 11.

**Table 11: Descriptive statistics of subjects who are ages 6 years or higher.**

| | | **Total** | **Study** | |
|---|---|---|---|---|
| | | | **005** | **007** |
| **Number of subjects** | | 77 | 17 | 60 |
| **# of HACs** | **Pre-study** | 25 | 8 | 17 |
| | **Post-study** | 15 | 3 | 12 |
| **Subjects with 1 or more HACs** | **Pre-study** | 15 | 5 | 10 |
| | **Post-study** | 12 | 3 | 9 |
| **Mean age at baseline (SD)** | | 24.7 (14.7) | 10.0 (3.5) | 28.9 (13.9) |
| **Mean baseline ammonia (µmol/L) (SD)** | | 27.6 (18.2) | 27.7 (12.4) | 27.5 (19.6) |
| **Baseline ammonia (µmol/L)** | **0** - **<0.5 ULN (% of subjects)** | 29 (37.7%) | 5 (29.4%) | 24 (40.0%) |
| | **0.5 - <1.0 ULN (% of subjects)** | 28 (36.3%) | 10 (58.8%) | 18 (30.0%) |
| | **≥1.0 ULN (% of subjects)** | 20 (26.0%) | 2 (11.8%) | 18 (30.0%) |
| **Baseline blood urea nitrogen** | **N** | 74 | 17 | 57 |
| | **Mean (SD)** | 2.84 (1.36) | 2.71 (1.04) | 2.87 (1.45) |
| **Baseline glutamine** | **N** | 73 | 17 | 56 |
| | **Mean (SD)** | 759.4 (250.2) | 694.9 (220.5) | 779.0 (257.1) |
| **Gender %** | **Male** | 28.6 | 17.7 | 31.7 |
| | **Female** | 71.4 | 82.3 | 68.3 |
| **Race %** | **White** | 80.5 | 82.4 | 80.0 |
| | **Non-white** | 19.5 | 17.6 | 20.0 |

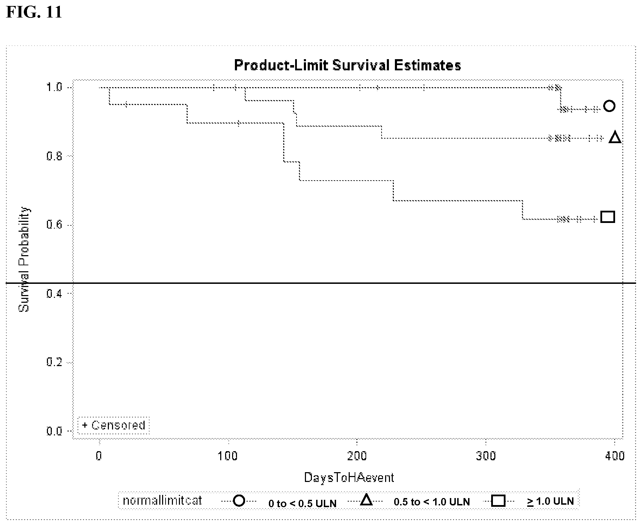

A KM plot illustrating time to first HAC in the 6 years and older subpopulation is shown in FIG. 11. This data showed a highly significant association between baseline fasting ammonia ULN categories and time to first HAC. A multivariate Cox proportional hazards model was generated that adjusted for age, gender, and race in the 6 years and older subpopulation. The results of this model are summarized in Table 12.

**Table 12: KM plot of time to first HAC by ULN baseline ammonia categories for subjects ages 6 years and older.**

| **Risk factor** | | **N** | **Hazard ratio** | **p-value** |
|---|---|---|---|---|
| **Age in years** | **Pediatric (6-10 years)** | 17 | Ref | |
| | **Pediatric (11-18 years)** | 9 | 0.21 | 0.192 |
| | **Adult (≥18 years)** | 51 | 0.17 | 0.055 |
| **Baseline ammonia (vs. ULN)** | **0 - <0.5 ULN** | 29 | Ref | Ref |
| | **0.5 - <1.0 ULN** | 28 | 2.97 | 0.337 |
| | **≥1.0 ULN** | 20 | 20.3 | 0.009 |
| **Gender (%)** | **Male** | 22 | 2.14 | 0.283 |
| | **Female** | 55 | Ref | Ref |
| **Race (%)** | **White** | 62 | 2.04 | 0.380 |
| | **Non-white** | 15 | Ref | Ref |

There was a significant association between baseline ammonia ULN categories and time from baseline to first HAC for patients who were at least 6 years of age at baseline (p = 0.0058) (FIG. 11).

After adjusting for age, gender, and race, a significant effect was still observed between baseline ammonia versus ULN and HAC risk. When patients < 6 years old at baseline (who experienced the highest rate of HACs and for whom measurement of fasting ammonia is problematic) were excluded, the increased risk of HAC with high baseline ammonia levels was even more apparent. Specifically, subjects age 6 and older had 3 times higher rate of HAC if their baseline fasting ammonia was between 0.5 to < 1 ULN and 20 times higher rate if their baseline ammonia levels were at or above 1.0 ULN than subjects with baseline ammonia levels < 0.5 (p=0.009). These results suggest that patients whose fasting ammonia levels are kept below 0.5 ULN are at the lowest risk of having a HAC and that fasting ammonia in subjects age 6 and older can be used as a predictor of risk of HAC.

### Example 6: Relationship between ammonia exposure and relative risk of HAC.

The relationship between total ammonia exposure over time and the risk of HAC occurrence was explored by calculating time normalized area under the curve (TNAUC₁₂ₘₒₙₜₕₛ) for ammonia for patients with and without an HAC during the 12-month treatment with GPB (Table 13). The AUC for ammonia, measured monthly, was calculated for each patient using the trapezoid rule over the course of the 12 month study. To normalize the AUC, TNAUCₓₘₒₙₜₕ, AUC was divided by the final study month of an ammonia assessment. The ammonia exposure considered either during the entire 12 months follow up or at any 3-months interval was higher in patients who experienced an HAC than those who did not.

**Table 13: TNAUC₁₂ₘₒₙₜₕₛ ammonia by HAC event.**

| | **HAC** | **No HAC** | **P-value** |
|---|---|---|---|
| **Month 0-3** | | | |
| **N** | 19 | 81 | |
| **Mean (SD)** | 33.3 (15.23) | 23.5 (13.62) | 0.0071 |
| **# HAC (% of Total HAC)** | 5 (18.5%) | | |
| **Incidence Rate/month** | 0.0170 | | |

| **Month 0-6** | | | |
|---|---|---|---|
| **N** | 19 | 81 | |
| **Mean (SD)** | 33.0 (12.17) | 24.0 (13.85) | 0.0100 |
| **# HAC (% of Total HAC)** | 13 (48.1%) | | |
| **Incidence Rate/month** | 0.0224 | | |

| **Month 0-9** | | | |
|---|---|---|---|
| **N** | 19 | 81 | |
| **Mean (SD)** | 33.4 (13.03) | 23.7 (12.56) | 0.0032 |
| **# HAC (% of Total HAC)** | 20 (74.1%) | | |
| **Incidence Rate/month** | 0.0234 | | |

| **Month 0-12** | | | |
|---|---|---|---|
| **N** | 19 | 81 | |
| **Mean (SD)** | 35.0 (14.99) | 23.6 (11.94) | 0.0006 |
| **# HAC (% of Total HAC)** | 27 (100%) | | |
| **Incidence Rate/month** | 0.0240 | | |
| Note: Cumulative Incidence=# HAC/persons at risk at beginning of interval. | | | |

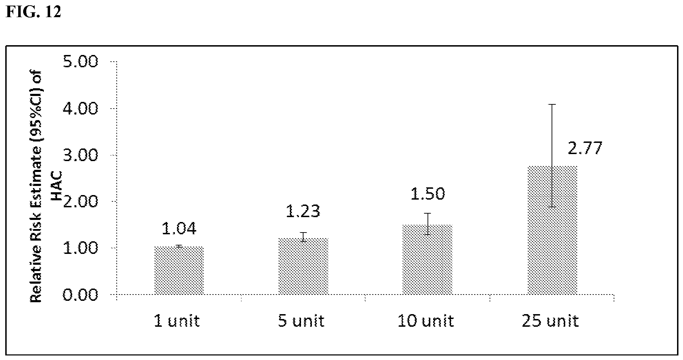

To further quantify the association of occurrence of HAC with the increase in ammonia level, the relative risk of experiencing an HAC per incremental increase in total ammonia exposure over 12 months (time normalized area under the curve of ammonia (TNAUC)) was also explored by using a Poisson regression method with a robust error variance (see Table 14 and FIG. 12). TNAUC over 12-months is correlated with the risk of experiencing an HAC at any time during that period.

**Table 14: Relative Risk (RR) of HAC events based on TNAUC ammonia.**

| **TNAUC₁₂ₘₒₙₜₕₛ** | **Relative Risk Estimate** | **Standard Error** | **Relative Risk** | | **Pvalue** |
|---|---|---|---|---|---|
| | | | **Confidence Limits** | | |
| **1 unit increase** | 1.0416 | 0.0082 | 1.0257 | 1.0578 | <0.0001 |
| **5 unit increase** | 1.2263 | 0.0482 | 1.1354 | 1.3245 | |
| **10 unit increase** | 1.5039 | 0.1182 | 1.2892 | 1.7544 | |
| **25 unit increase** | 2.7736 | 0.5450 | 1.8870 | 4.0766 | |
| HAC: hyperammonemic crisis; TNAUC: time-normalized area under the curve. | | | | | |

For a 1-unit increase in ammonia, the relative risk (95% CI) was 1.0416 (1.0257-1.0578) and increased to 1.2263 (1.1354-1.325) for a 5-unit increase, which reflects a 4% and 23%, respectively, increase in risk of experiencing a HAC. Similarly increases of 10 and 25 µmol/L increased the relative risk of HAC by 50% and 270%, respectively (p<0.0001 for all comparisons) (Table 14).

### Example 7: Relationship between study site and baseline ammonia levels within each ULN category.

The proportion of patients with baseline ammonia levels within each ULN category varied considerably among study sites (FIG. 13A), which were therefore grouped into three categories based on the percentage of patients enrolled whose baseline fasting ammonia was < 0.5 of the ULN at baseline: Group 1 (6 study sites; 33 patients, of whom >50% had baseline ammonia levels < 0.5 ULN); Group 2 (3 study sites; 29 patients, of whom 25%-50% had baseline ammonia levels < 0.5 ULN); and Group 3 (11 sites; 38 patients, of whom < 25% had baseline ammonia < 0.5 ULN) (FIG. 13B and Table 15).

**Table 15: Demographics of patients among study sites in relation to ammonia control.**

| | **Grouping of study sites based on % of subjects whose ammonia at baseline was less than half ULN** | | |
|---|---|---|---|
| | **Group 1** | **Group 2** | **Group 3** |
| **Number of sites** | 6 | 3 | 11 |
| **Number of subjects** | 33 | 29 | 38 |

| **Age at baseline** | | | |
|---|---|---|---|
| **Mean in years (SD)** | 24.5 (19.8) | 20.5 (13.2) | 14.7 (12.4) |

| **Age at baseline, N (%)** | | | |
|---|---|---|---|
| **<=2 years** | 2(6.1) | 3 (10.3) | 5 (13.2) |
| **>2 years** | 31 (93.9) | 26 (89.7) | 33 (86.8) |

| **Baseline Ammonia (µmol/L)** | | | |
|---|---|---|---|
| **Mean (SD)** | 19.9 (20.3) | 26.9 (16.0) | 37.9 (18.8) |
| **% 0-<0.5 normal limit** | 72.7 | 37.9 | 10.5 |
| **% 0.5-<1.0normal limit** | 18.2 | 24.2 | 55.3 |
| **% ≥1 normal limit** | 9.1 | 37.9 | 34.2 |

| **Gender (%)** | | | |
|---|---|---|---|
| **Male** | 42.4 | 27.6 | 29.0 |
| **Female** | 57.6 | 72.4 | 71.0 |
| **Among Females only, N (%) OTC subtype Any other subtype** | 16 (84.2) 3 (15.2) | 17 (81.0) 4 (19.0) | 20 (74.1) 7 (25.9) |

| **Binary Subtype, N (%)** | | | |
|---|---|---|---|
| **OTC+Female** | 16 (48.8) | 17 (58.6) | 20 (52.6) |
| **Any other** | 17 (51.2) | 12(41.4) | 18(47.4) |

| **Subtype (%)** | | | |
|---|---|---|---|
| **OTC** | 66.7 | 69.0 | 71.1 |
| **ARG** | 0.0 | 3.5 | 2.6 |
| **ASL** | 21.2 | 10.3 | 7.9 |
| **ASS** | 9.1 | 6.9 | 18.4 |
| **CPS** | 0.0 | 3.5 | 0.0 |
| **HHH** | 3.0 | 6.9 | 0.0 |

| **Prescribed Protein (g/kg/day)** | | | |
|---|---|---|---|
| **Mean (SD)** | 0.92 (0.42) | 0.86 (0.47) | 0.97 (0.41) |
| *Study sites were grouped in relation to ammonia levels among patients enrolled at that site: *Group 1(6 study sites; 33 patients, of whom* > *50% had baseline ammonia levels* < *0.5 ULN); Group 2 (3 study sites; 29 patients, of whom 25%-50% had baseline ammonia levels* < *0.5 ULN); and Group 3 (11 sites; 38 patients, of whom* < *25% had baseline ammonia* < *0.5 ULN)* | | | |

The proportion of patients who experienced an HAC was lowest (12%) among Group 1 sites and increased progressively to 21% and 24% in Group 2 and 3 sites (FIG. 13B). Although the mean age of patients in Group 3 was lower compared to Group 1 (14.7 vs 24.5yrs), other markers of disease severity including the proportion of females with OTC deficiency and restriction of dietary protein were similar among the groups. Baseline characteristics including age, gender, and UCD subtype were similar across the ULN groups. Thus, the fact that lower fasting ammonia levels correlate strongly with lower risk and frequency of HAC does not likely reflect intrinsic differences in disease severity. Instead, the data pertaining to ammonia levels and frequency of HAC among patients enrolled at more than 20 sites in North America, even allowing for possible differences in compliance among patients at different sites, appear to be explained by different responses to management or differing management approaches rather than regional difference in severity of illness.

### Example 8: Relationship between glutamine levels and different ammonia categories

The relationship between glutamine levels and ammonia categories was also analyzed. Published data from Blau et al. indicates that the upper limit of normal glutamine values for different age categories is as follows: 4 months to 2 years of age (709 µmol/L), 2 to 10 years of age (709 µmol/L), 10 to 18 years of age (740 µmol/L), older than 18 years of age (721 µmol/L) (Blau 2008). Glutamine levels were analyzed over the 12 month study period in patients treated for 12 months with GPB. Monthly glutamine levels were generally higher in patients in the higher baseline ammonia ULN groups and, by three months of treatment, had decreased in the patients with the highest baseline glutamine at baseline (FIG. 14A).

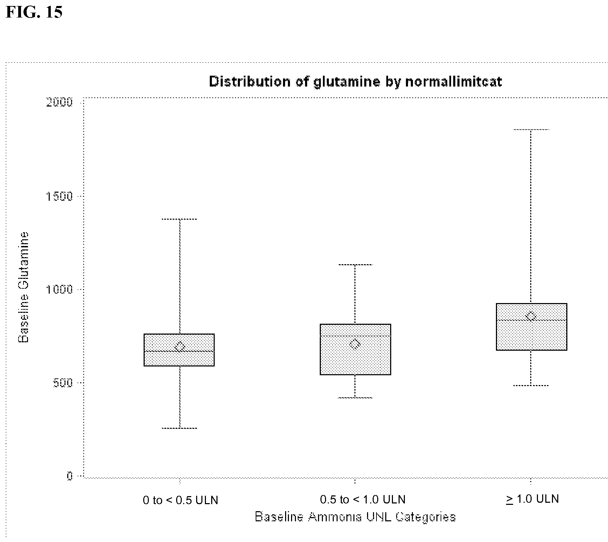

There was a statistically significant but comparatively weak correlation between baseline ammonia and glutamine levels at the time of enrollment (r = 0.27; p = 0.008) as well as between fasting glutamine levels and daily ammonia exposure on GPB or NaPBA during the switchover studies (r = 0.292, p<0.001). However, when the relationship between glutamine and ammonia categories with respect to the ULN was analyzed, glutamine values varied substantially for patients in each category and the overall correlation appeared to be driven by patients whose a baseline ammonia exceeded 1.0 ULN (FIGs. 14A and B). The descriptive statistics of baseline glutamine by upper limit categories are presented in FIG. 15 and Table 16.

**Table 16: Descriptive statistics of glutamine by ULN categories.**

| **ULN Category** | **N** | **Mean (µmol/L)** | **SD** | **Minimum** | **Maximum** |
|---|---|---|---|---|---|
| **0.0** - < **0.5** | 37 | 693.6 | 199.0 | 257.0 | 1378.0 |
| **0.5** - < **1.0** | 33 | 709.7 | 183.3 | 419.0 | 1135.0 |
| ≥ **1.0** | 25 | 856.5 | 306.0 | 483.0 | 1857.0 |

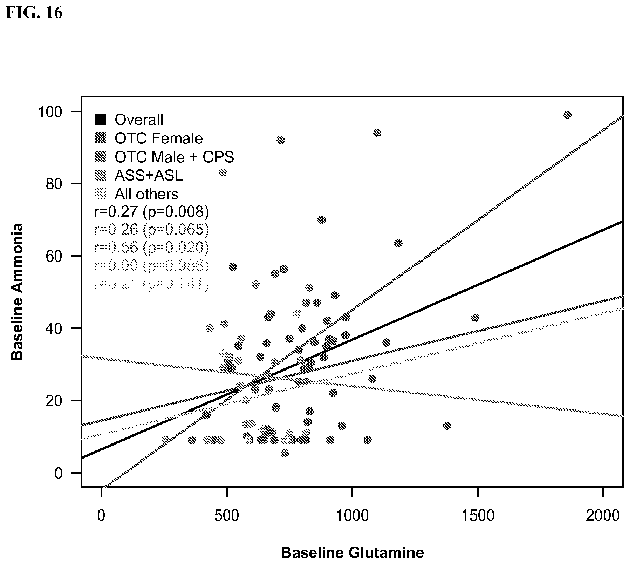

Additionally, patients with proximal deficiencies (OTC and CPS) showed a stronger correlation between glutamine and ammonia (FIG. 16). There was no correlation, however, between baseline ammonia and UCD subtype or levels of either citrulline (r=0.04; p=0.414) or argininoosuccinic acid (r=0.14; p=0.158).

An analysis was also performed to determine mean differences in glutamine levels in patients who experienced an HAC and those who did not (Table 17).

**Table 17: Mean comparison of glutamine by HAC.**

| **HAC** | **N** | **Mean (µmol/L)** | **SD** | **Minimum** | **Maximum** |
|---|---|---|---|---|---|
| **No** | 76 | 732.0 | 245.9 | 257.0 | 1857.0 |
| **Yes** | 19 | 782.4 | 186.2 | 433.0 | 1184.0 |

The mean glutamine was 50.0 units higher in subjects with an HAC compared to those without an HAC during the study (782.4 vs. 732.0 µmol/L; p = 0.150) or as total glutamine exposure (TNAUC₁₂ₘₒₙₜₕ of 720.9 vs. 699.4 µmοl/L^{∗}m; p=0.686).

A multivariable Cox regression analysis with baseline ULN ammonia values, baseline glutamine values, sex, age, and race was performed and is provided in Table 18.

**Table 18: Multivariable Cox regression results for time to first HAC during the study with the inclusion of glutamine.**

| **Risk Factor** | **N** | **Hazard Ratio** | **p-value** |
|---|---|---|---|
| **Age in years** | | | |
| **Pediatric: <18** | 46 | 3.12 | 0.034 |
| **Adult: ≥18** | 49 | Ref | Ref |

| **Baseline Ammonia (µmol/L)** | | | |
|---|---|---|---|
| **0- < 0.5 ULN** | 37 | Ref | Ref |
| **0.5- < 1.0 ULN** | 33 | 1.27 | 0.728 |
| **≥ 1.0 ULN** | 25 | 4.29 | 0.020 |

| **Glutamine Levels** | | | |
|---|---|---|---|
| **0- < 649 µmol/L** | 32 | Ref | Ref |
| **649-808 µmol/L** | 31 | 2.59 | 0.177 |
| **≥ 809 µmol/L** | 32 | 2.79 | 0.140 |

| **Gender (%)** | | | |
|---|---|---|---|
| **Male** | 31 | 2.21 | 0.121 |
| **Female** | 64 | Ref | Ref |

| **Race (%)** | | | |
|---|---|---|---|
| **White** | 77 | 1.34 | 0.623 |
| **Non-white** | 18 | Ref | Ref |
| Note, that N= 95 because 5 subjects did not have glutamine values at baseline. | | | |

As shown in Table 18, after controlling for the addition of glutamine levels, ULN ammonia categories continued to have a significant association with the risk of HAC. Additionally, an elevated risk of HAC was observed with an increase in glutamine levels. Specifically, patients had about a 2.5 times higher rate of risk of first HAC if their glutamine levels were between 649 µmol/L to 808 µmol/L and about a 2.8 times higher rate of risk of first HAC if their glutamine levels were ≥ 809 µmol/L compared to patients with glutamine levels less than 649 µmol/L.

Glutamine levels were slightly higher at baseline in patients who experienced an HAC compared to those who did not experience an HAC during the study. Glutamine correlated modestly with fasting ammonia at baseline and decreased during GPB treatment in patients with the highest baseline glutamine levels. Interestingly, the correlation between fasting ammonia and glutamine seemed to be primarily driven by patients with the highest ammonia. This may explain the apparent discrepancy between the present findings and those reported by Maestri et al, whose patient exhibited higher ammonia values than did those patients in the present analyses (Maestri 1992).

### REFERENCES

1. Blau Laboratory Guide to the Methods in Biochemical Genetics 71 (2008)
2. Brusilow Science 207:659 (1980)
3. Brusilow Pediatr Res 29:147 (1991)
4. Diaz Mol Genet Metab 102:276 (2011)
5. Ghabril Gastroenterology 142:S918 (2012)
6. Haberle Eur. J. Pediatr. 170:21 (2011)
7. Haberle Orphanet J Rare Dis 7:1750 (2012)
8. Lee Mol Genet Metab 100:221 (2010)
9. Lichter-Konecki Mol Genet Metab 103:323 (2011)
10. McGuire Hepatology 51:2077 (2010)
11. Maestri J. Pediatr. 121:259 (1992)
12. Rockey Hepatology 56:248 (2012)
13. Kaplan Journal of the American Statistical Association 53: 457 (1958)
14. Peto Journal of the Royal Statistical Society 185 (1972)
15. Hardin Generalized Estimating Equations (2005)

## Claims

1. Glycerol phenylbutyrate (GPB) for use in a method of preventing a hyperammonemic crisis (HAC) in a subject with a urea cycle disorder (UCD) who has been previously treated with a nitrogen scavenging drug and who has a glutamine level greater than or equal to 649 µmol/L, wherein the method comprises:
(a) measuring a fasting blood ammonia level;
(b) comparing the fasting blood ammonia level to the upper limit of normal (ULN) for blood ammonia; and
(c) administering an effective dosage of GPB to the subject if the fasting blood ammonia level is above 0.5 times the ULN for fasting blood ammonia,
wherein the effective dosage of GPB results in the fasting blood ammonia level being <0.5 times the ULN for blood ammonia,
wherein the subject is aged 6 years or older, and
wherein the nitrogen scavenging drug is selected from the group consisting of sodium benzoate, GPB, phenylbutyric acid (PBA), NaPBA, and a combination of two or more of GPB, PBA, and NaPBA.

2. GPB for use as in claim 1, wherein the effective dosage of GPB results in the fasting blood ammonia level being <0.5 times the ULN for blood ammonia after multiple administrations.

3. GPB for use as in claim 1, wherein the subject has previously been administered a first dosage of GPB, preferably
wherein the dosage of GPB administered in step (c) is greater than the first dosage.

4. GPB for use as in claim 1, wherein the effective dosage of GPB is administered to the subject if the fasting blood ammonia level is equal to or greater than 1.0 ULN and the patient is older than 6 years old.

5. GPB for use as in any of claims 1-3, wherein the HAC is a first HAC.

6. GBP for use as in any one of claims 1-5, wherein the subject has been previously treated with GPB for an amount of time to maintain a steady state level of the drug in the subject.

7. GBP for use as in claim 1, wherein the subject has not been previously treated with GPB, preferably
wherein the subject has been previously treated with sodium phenylbutyrate (NaPBA).

## Patentansprüche

1. Glycerolphenylbutyrat (GPB) zur Anwendung in einem Verfahren zur Verhinderung einer hyperammonämischen Krise (HAC) in einem Individuum mit einer Harnstoffzykluserkrankung (UCD), das vorhergehend mit einem Stickstoffbindungsmedikament behandelt wurde und einen Glutaminspiegel größer oder gleich 649 µmol/L aufweist, wobei das Verfahren Folgendes umfasst:
(a) Messen eines Blutammoniakspiegels unter Fasten;
(b) Vergleichen des Blutammoniakspiegels unter Fasten mit der oberen Normgrenze (ULN) für Blutammoniak; und
(c) Verabreichen einer wirksamen Dosierung von GPB an das Individuum, wenn der Blutammoniakspiegel über 0,5-mal so hoch ist wie die ULN für Blutammoniak unter Fasten,
wobei die wirksame Dosierung von GPB zu einem Blutammoniakspiegel unter Fasten von <0,5-mal der ULN für Blutammoniak führt,
wobei das Individuum 6 Jahre oder älter ist, und
wobei das Stickstoffbindungsmedikament gewählt ist aus der Gruppe bestehend aus Natriumbenzoat, GPB, Phenylbuttersäure (PBA), NaPBA und einer Kombination von zwei oder mehreren von GPB, PBA und NaPBA.

2. GPB zur Anwendung nach Anspruch 1, wobei die wirksame Dosierung von GPB nach mehreren Verabreichungen zu einem Blutammoniakspiegel unter Fasten von <0,5-mal der ULN für Blutammoniak führt.

3. GPB zur Anwendung nach Anspruch 1, wobei dem Individuum vorhergehend eine erste Dosierung von GPB verabreicht wurde, vorzugsweise wobei die in Schritt (c) verabreichte Dosierung von GPB größer ist als die erste Dosierung.

4. GPB zur Anwendung nach Anspruch 1, wobei die wirksame Dosierung von GPB an das Individuum verabreicht wird, wenn der Blutammoniakspiegel unter Fasten gleich oder größer als 1,0 ULN ist und der Patient älter als 6 Jahre ist.

5. GPB zur Anwendung nach einem der Ansprüche 1 bis 3, wobei die HAC eine erste HAC ist.

6. GBP zur Anwendung nach einem der Ansprüche 1 bis 5, wobei das Individuum vorhergehend über einen Zeitraum mit GPB behandelt wurde, um einen stabilen Zustandsspiegel des Medikaments im Individuum beizubehalten.

7. GBP zur Anwendung nach Anspruch 1, wobei das Individuum nicht vorhergehend mit GPB behandelt wurde, vorzugsweise
wobei das Individuum vorhergehend mit Natriumphenylbutyrat (NaPBA) behandelt wurde.

## Revendications

1. Phénylbutyrate de glycérol (GPB) à utiliser dans un procédé de prévention d'une crise hyperammoniologique (HAC) chez un sujet présentant un trouble du cycle de l'urée (UCD) qui a été traité précédemment avec un médicament épurateur d'azote et qui présente un taux de glutamine supérieur ou égal à 649 µmol/L, dans lequel le procédé comprend les étapes consistant à :
(a) mesurer un taux d'ammoniac dans le sang à jeun;
(b) comparer le taux d'ammoniac dans le sang à jeun à la limite supérieure de la normale (ULN) pour l'ammoniac dans le sang; et
(c) administrer une dose efficace du GPB au sujet si le taux d'ammoniac dans le sang à jeun est supérieur à 0,5 fois l'ULN pour la quantité d'ammoniac dans le sang à jeun,
dans lequel la dose efficace du GPB aboutit au taux d'ammoniac dans le sang à jeun
étant <0,5 fois l'ULN pour une quantité d'ammoniac dans le sang,
dans lequel le sujet a 6 ans ou plus, et
dans lequel le médicament épurateur d'azote est choisi dans le groupe consistant en benzoate de sodium, GPB, acide phénylbutyrique (PBA), NaPBA, et d'une combinaison de deux ou plus de GPB, de PBA et de NaPBA.

2. GPB pour l'usage selon la revendication 1, dans lequel la dose efficace du GPB aboutit au taux d'ammoniac dans le sang à jeun étant <0,5 fois l'ULN pour la quantité d'ammoniac dans le sang après multiples administrations.

3. GPB pour l'usage selon la revendication 1, dans lequel une première dose du GPB est précédemment administrée au sujet, de préférence dans lequel la dose du GPB administrée dans l'étape (c) est supérieure à la première dose.

4. GPB pour l'usage selon la revendication 1, dans lequel la dose efficace du GPB est administrée au sujet si le taux d'ammoniac dans le sang à jeun est égal ou supérieur à 1,0 ULN et le sujet a 6 ans ou plus.

5. GPB pour l'usage selon l'une quelconque des revendications 1 à 3, dans lequel le HAC est un premier HAC.

6. GPB pour l'usage selon l'une quelconque des revendications 1 à 5, dans lequel le sujet a été traité précédemment avec du GPB pendant une durée suffisante pour maintenir un taux du médicament à l'état d'équilibre du sujet.

7. GPB pour l'usage selon la revendication 1, dans lequel le sujet n'a pas été traité précédemment avec du GPB, de préférence
dans lequel le sujet a été traité précédemment avec du sodium phenylbutyrate (NaPBA).
